# EUROPEAN PATENT SPECIFICATION

(11) **EP 3 307 233 B1**
(45) Date of publication and mention of the grant of the patent: **06.11.2019**
(21) Application number: 16728043.7
(22) Date of filing: 10.06.2016
(51) Int. Cl.: A61K 8/37, A61K 8/49, A61Q 17/04, A61K 8/81, A61K 8/891, A61K 8/893, A61K 8/06

(54) **COMPOSITION COMPRISING A UV-SCREENING AGENT, AN ANIONIC CROSSLINKED HYDROPHILIC POLYMER, A SURFACTANT HAVING AN HLB LESS THAN OR EQUAL TO 5 AND A SILICONE COPOLYMER**
ZUSAMMENSETZUNGEN MIT EINEM UV-SCHUTZ-MITTEL, EINEM HYDROPHILEN VERNETZTEN ANIONISCHEN POLYMER, EINEM TENSID MIT EINEM HLB-WERT GRÖSSER ALS ODER GLEICH ALS 5 UND EINEM SILIKONCOPOLYMER
COMPOSITIONS COMPRENANT UN FILTRE UV, UN POLYMERE HYDROPHILE RETICULE ANIONIQUE, UN TENSIOACTIF AYANT UNE HLB INFERIEURE OU EGALE A 5 ET UN COPOLYMERE SILICONE.

(30) Priority: 11.06.2015 FR 1555320; 15.06.2015 US 201562175635 P
(43) Date of publication of application: 18.04.2018
(73) Proprietor: L'Oréal, 75008 Paris (FR)
(72) Inventor: L'ALLORET, Florence, 75014 Paris (FR); GROC-BIDAULT, Marina, 94160 Saint-mande (FR); CZIRYAK, Paula, Eatontown, New Jersey 07724 (US)
(74) Representative: Casalonga
(86) International application number: PCT/EP2016/063268
(87) International publication number: WO 2016/198581

(56) References cited:
- EP-A1- 2 853 255
- EP-A2- 2 425 810
- WO-A2-2007/140442
- US-A- 5 830 447
- US-A1- 2007 178 057
- US-A1- 2013 078 204
- Isabelle Van Reeth ET AL: "Silicones Bring Multi-functional Performance to Sun Care", Cosmetics and Toiletries, 1 January 2006 (2006-01-01), pages 351-355, XP055162957, Retrieved from the Internet: URL:http://www.alluredbooks.com/sample_pag es/poly_path_vers_tech_ch26.pdf [retrieved on 2015-01-19]

## Description

The present invention relates to a composition as defined in the appended claims in the form of an oil-in-water emulsion comprising one or more UV-screening agents, one or more anionic crosslinked hydrophilic polymers, one or more surfactants having an HLB less than or equal to 5 and one or more specific silicone copolymers.

The present invention also relates to a composition as defined in the appended claims for its use in treating the skin against UV radiation using said composition.

Light radiation with a wavelength of between 280 and 400 nm makes it possible to brown human epidermis. Rays with a wavelength of between 280 and 320 nm, known as UVB rays, cause skin erythema and burns which can be detrimental to the development of a tan. Rays with a wavelength of between 320 and 400 nm, known as UVA rays, are capable of bringing about a detrimental change in the skin, with in particular a loss of elasticity and the appearance of wrinkles, resulting in premature ageing.

UV rays must therefore be screened out. Currently, there exist protective cosmetic compositions for the human epidermis containing UV-screening agents which are active with regard to screening out UVA and UVB radiation.

The conditions for using an anti-sun composition are extremely varied and depend on the activities performed by the consumer. Use on the beach is very common, thereby resulting in applications both to dry skin and to wet skin, in particular after swimming.

However, on wet skin, active substances, such as UV-screening agents, have a tendency to become diluted, impairing their skin protection properties.

Among the various textures of anti-sun compositions that exist, the majority of them are difficult to apply to wet skin. This is because the spreading of the composition on wet skin brings about a considerable whitening effect, which is not very pleasant for the consumer. Very lengthy massaging of the composition is then required in order to obtain a uniform appearance. This is in particular the case with milk textures, which are widely appreciated moreover for their qualities in terms of application to dry skin.

Currently, alcoholic transparent compositions, generally provided in a pump-spray packaging, correspond to the compositions that are the most compatible with application to wet skin. The whitening effect on the skin and the application are acceptable, but do not however correspond to good qualities with regard to use on wet skin. Their anti-sun protection properties are not entirely satisfactory.

Moreover, these compositions comprise substantial alcohol contents and consequently have many drawbacks. They cannot, for example, be used on the face and can cause a drying out effect on the skin.

Thus, there is a real need to make available an anti-sun composition which does not have the drawbacks mentioned above, that is to say a milk-type anti-sun composition which is stable, which is compatible with application to wet skin, and the *in vivo* sun protection factor (SPF) level of which is high.

The applicant has discovered, surprisingly, that a composition in the form of an oil-in-water emulsion comprising one or more UV-screening agents, one or more anionic crosslinked hydrophilic polymers, one or more surfactants having an HLB less than or equal to 5 and one or more specific silicone copolymers makes it possible to achieve the objectives set out above, in particular to obtain a screening composition with a high SPF and having an excellent compatibility with application to wet skin.

A subject of the present invention is in particular a composition in the form of an oil-in-water emulsion comprising:
a) an oily phase, dispersed in a continuous aqueous phase,
b) one or more UV-screening agents chosen from water-soluble organic UV-screening agents, liposoluble organic UV-screening agents, insoluble organic UV-screening agents, and mixtures thereof
c) one or more anionic crosslinked hydrophilic polymers,
d) one or more surfactants having an HLB less than or equal to 5, and
e) one or more silicone copolymers resulting from the copolymerization of at least one monomer of the type of an ester of a carboxylic acid and of an alcohol comprising a C₆ to C₃₀ fatty chain, with at least one monomer containing a polyalkylsiloxane chain;
the weight ratio between the amount of silicone copolymer(s) and the amount of anionic crosslinked hydrophilic polymer(s) is less than or equal to 5.

Contrary to the conventional anti-sun compositions, the composition of the invention makes it possible to combine a satisfactory application to wet skin with a high sun protection.

Indeed, the composition of the invention exhibits good use properties. It spreads easily and allows uniform application without the appearance of a whitish film on the skin.

The composition also makes it possible to filter out UV rays at a high SPF, consequently leading to better skin protection.

The composition of the invention is also stable over time.

Moreover, the composition of the invention has good cosmetic properties, in particular in terms of tacky-feel effect. It is in fact pleasant and not very tacky.

A subject of the present invention is also a composition as defined above for its use in treating the skin against UV radiation, in particular against solar radiation.

Other subjects, characteristics, aspects and advantages of the invention will become even more clearly apparent on reading the description and the examples which follow.

In the text hereinbelow, and unless otherwise indicated, the limits of a range of values are included in that range, in particular in the expressions "between" and "ranging from ... to ...".

Moreover, the expressions "at least one" and "at least" used in this description are equivalent to the expressions "one or more" and "greater than or equal" respectively.

For the purposes of the present patent application, the term "(meth)acrylic" and "(meth)acrylate" are intended to mean acrylic or methacrylic and acrylate or methacrylate.

For the purposes of the present application, the term "high SPF" is intended to mean an *in vitro* sun protection factor (SPF) which can reach a value of at least 100, and preferably of at least 130. The level of sun protection is defined by the sun protection factor (SPF) which is determined according to the *"in vitro"* method described by B. L. Diffey in J. Soc. Cosmet. Chem. 40, 127-133, (1989).

According to the present invention, the term "stable over time" is intended to mean a composition which, after one month, preferably after two months, of storage at a temperature ranging from 4°C to 45°C, does not exhibit any macroscopic change in colour, in odour or in viscosity, nor any variation in pH, and also no variation in microscopic appearance.

For the purposes of the present invention, the term "good compatibility on wet skin" is intended to mean that on application of the composition in a proportion of 2mg.cm⁻², to a pre-wetted skin substitute substrate, a uniform film which is transparent to the naked eye is obtained.

### UV-screening agents

The composition according to the present invention comprises one or more UV-screening agents.

According to one preferred embodiment, the UV-screening agent(s) are included in the oily phase of the composition.

According to the present invention, the expression "UV-screening agents" is equivalent to the expression "photoprotective agent".

The UV-screening agent(s) that are suitable for the present invention are chosen from water-soluble organic UV-screening agents, liposoluble organic UV-screening agents, insoluble organic UV-screening agents, and mixtures thereof. The UV-screening agent(s) are chosen preferentially from liposoluble organic UV-screening agents, and mixtures thereof.

The term "water-soluble organic UV-screening agent" is intended to mean any organic compound for screening out UV radiation which can be fully dissolved in molecular form or miscible in a liquid aqueous phase or else can be dissolved in colloidal form (for example in micellar form) in a liquid aqueous phase.

The term "liposoluble organic UV-screening agent" is intended to mean any cosmetic or dermatological organic compound for screening out UV radiation which can be fully dissolved in molecular form or miscible in an oily phase or else can be dissolved in colloidal form (for example in micellar form) in an oily phase.

The term "insoluble organic UV-screening agent" is intended to mean any cosmetic or dermatological organic compound for screening out UV radiation which has a solubility in water of less than 0.5% by weight and a solubility of less than 0.5% by weight in the majority of organic solvents such as liquid paraffin, fatty alcohol benzoates and fatty acid triglycerides, for example Miglyol 812® sold by the company Dynamit Nobel. This solubility, determined at 70°C, is defined as the amount of product in solution in the solvent at equilibrium with an excess of solid in suspension after returning to ambient temperature. It may be readily evaluated in the laboratory.

The composition according to the present invention may comprise one or more water-soluble organic UV-screening agents chosen from water-soluble organic UVA-screening agents, water-soluble organic UVB-screening agents, and mixtures thereof.

The term "water-soluble organic UVA-screening agent" is intended to mean any organic compound for screening out UVA radiation in the wavelength range 320 to 400 nm which can be fully dissolved in molecular form or miscible in a liquid aqueous phase or else can be dissolved in colloidal form (for example in micellar form) in a liquid aqueous phase.

Among the water-soluble organic UVA-screening agents that can be used according to the present invention, mention may be made of benzene-1,4-di(3-methylidene-10-camphorsulfonic) acid (INCI name: Terephthalylidene Dicamphor Sulfonic Acid) and the various salts thereof, described in particular in patent applications FR-A-2528420 and FR-A-2639347.

These screening agents correspond to general formula (I) below: 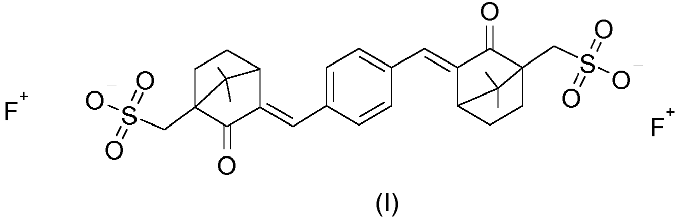 in which F denotes a hydrogen atom, an alkali metal or else an NH(R₁)₃⁺ radical in which the R₁ radicals, which may be identical or different, denote a hydrogen atom, a C₁ to C₄ alkyl or hydroxyalkyl radical or else an M^{n+ /}n group, Mn+ denoting a polyvalent metal cation in which n is equal to 2 or 3 or 4, Mn+ preferably denoting a metal cation chosen from Ca²⁺, Zn²⁺, Mg²⁺, Ba²⁺, Al³⁺ and Zr⁴⁺. It is clearly understood that the compounds of formula (I) above can give rise to the "cis-trans" isomer around one or more double bond(s) and that all the isomers are within the context of the present invention.

Among the hydrophilic organic UVA-screening agents that can be used according to the present invention, mention may also be made of compounds comprising at least two benzazolyl groups bearing sulfonic groups, such as those described in patent application EP-A-0669323.

They are described and prepared according to the syntheses indicated in patent US 2 463 264 and also in patent application EP-A-0669323.

The compounds comprising at least two benzazolyl groups in accordance with the invention correspond to general formula (II) below: 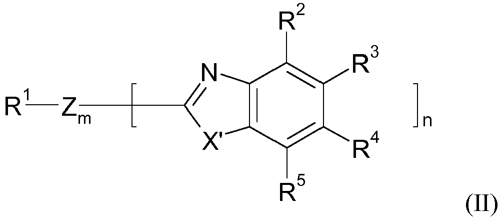 in which
- Z represents an organic residue of valency (1 + n) comprising one or more double bonds placed such that it completes the system of double bonds of at least two benzazolyl groups as defined inside the square brackets so as to form a totally conjugated assembly;
- X' denotes S, O or NR⁶;
- R¹ denotes a hydrogen atom, a C₁ to C₁₈ alkyl, a C₁ to C₄ alkoxy, a C₅ to C₁₅ aryl, a C₂ to C₁₈ acyloxy, or an SO₃Y or COOY group;
- the R², R³, R⁴ and R⁵ radicals, which may be identical or different, denote a nitro group or an R¹ radical;
- R⁶ denotes a hydrogen atom, a C₁ to C₄ alkyl or a C₁ to C₄ hydroxyalkyl;
- Y denotes hydrogen, Li, Na, K, NH₄, 1/2Ca, 1/2Mg, 1/3Al or a cation resulting from the neutralization of a free acid group with an organic nitrogenous base;
- m is 0 or 1;
- n is a number from 2 to 6;
- l is a number from 1 to 4;
- with the proviso that 1 + n does not exceed the value 6.

Among these compounds, preference is given to those for which the group Z is chosen from the group made up of:
(a) an olefin linear aliphatic C₂ to C₆ hydrocarbon-based radical which may be interrupted with a C₅ to C₁₂ aryl group or a C₄ to C₁₀ heteroaryl group, in particular chosen from the following groups: 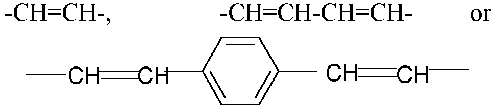
(b) a C₅ to C₁₅ aryl group which may be interrupted with an olefin linear aliphatic C₂ to C₆ hydrocarbon-based radical, in particular chosen from the following groups: 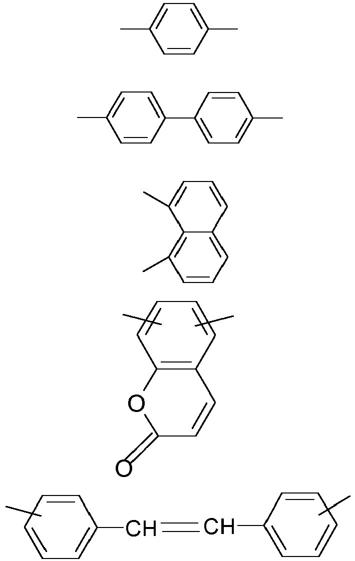
(c) a C₃ to C₁₀ heteroaryl residue, in particular chosen from the following groups: 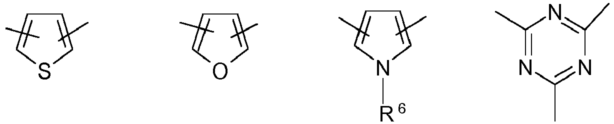 wherein R⁶ as the same meaning as that indicated above; said Z radicals as defined in paragraphs (a), (b) and (c) possibly being substituted with C₁ to C₆ alkyl, C₁ to C₆ alkoxy, phenoxy, hydroxyl, methylenedioxy or amino radicals optionally substituted with one or 2 C₁ to C₅ alkyl radicals.

Preferably, the compounds of formula (II) comprise, per molecule, 1, 3 or 4 SO₃Y groups.

By way of examples of compounds of formula (II) that can be used, mention may be made of the compounds of formulae (a) to (j) having the following structures, and also the salts thereof:

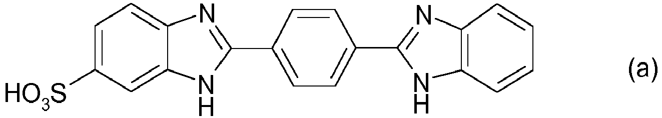

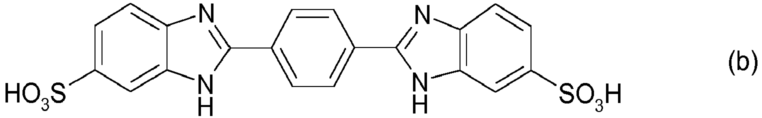

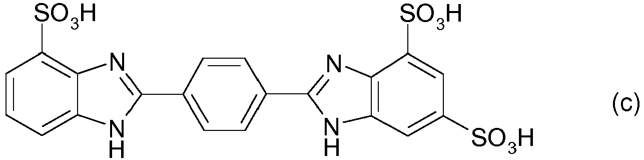

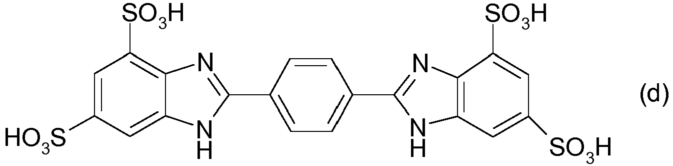

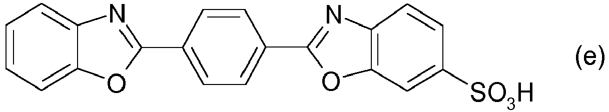

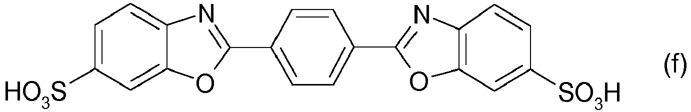

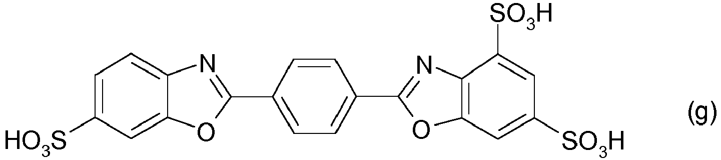

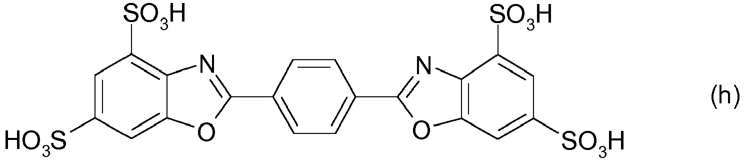

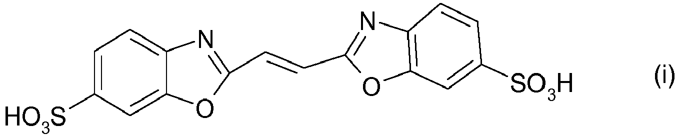

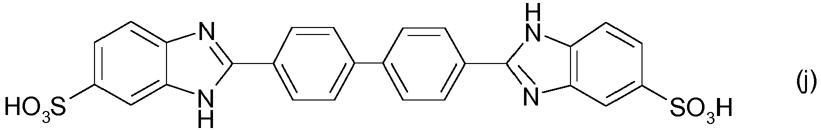

Among all these compounds, preference will most particularly be given to 1,4-bis-benzimidazolylphenylene-3,3',5,5'-tetrasulfonic acid (INCI name: Disodium Phenyl Dibenzimidazole Tetrasulfonate) (compound (d)) or a salt thereof, having the following structure, sold in particular under the name Neoheliopan AP® by the company Symrise:

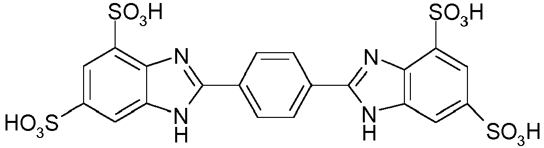

Among the water-soluble organic UVA-screening agents that can be used according to the present invention, mention may also be made of benzophenone compounds comprising at least one sulfonic acid function, for instance the following compounds:
- Benzophenone-4, sold in particular by the company BASF under the name Uvinul MS40® 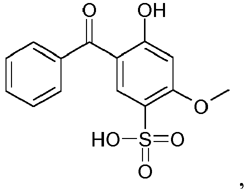
- Benzophenone-5 having the structure 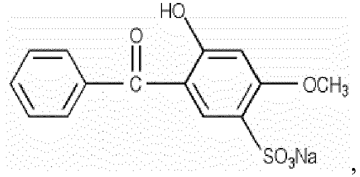
- Benzophenone-9, sold in particular by the company BASF under the name Uvinul DS49®

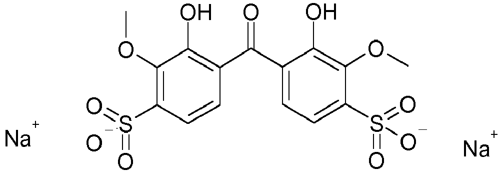

Among the water-soluble organic UVA-screening agents, use will more particularly be made of benzene-1,4-di(3-methylidene-10-camphorsulfonic) acid and the various salts thereof (INCI name: Terephthalylidene Dicamphor Sulfonic Acid) produced by the company Chimex under the trade name Mexoryl SX®.

The water-soluble organic UVB-screening agents that can be used according to the present invention are in particular chosen from water-soluble cinnamic derivatives, such as ferulic acid or 3-methoxy-4-hydroxycinnamic acid; water-soluble benzylidenecamphor compounds; water-soluble phenylbenzimidazole compounds; water-soluble p-aminobenzoic (PABA) compounds; water-soluble salicylic compounds, and mixtures thereof.

As examples of water-soluble organic UVB-screening agents, mention may be made of the following compounds denoted under their INCI name:
- para-aminobenzoic compounds, such as PABA, or PEG-25 PABA sold in particular under the name Uvinul P 25® by BASF;
- salicylic compounds, such as dipropylene glycol salicylate sold in particular under the name Dipsal® by Scher, and TEA salicylate, sold in particular under the name Neo Heliopan TS® by Symrise;
- benzylidenecamphor compounds, such as benzylidenecamphorsulfonic acid sold in particular under the name Mexoryl SL® by Chimex, and camphor benzalkonium methosulfate sold in particular under the name Mexoryl SO® by Chimex;
- phenylbenzimidazole compounds, such as phenylbenzimidazolesulfonic acid, sold in particular under the trade name Eusolex 232® by Merck.

Use will more particularly be made of the screening agent phenylbenzimidazolesulfonic acid, sold in particular under the trade name Eusolex 232® by Merck.

The composition according to the present invention may comprise one or more liposoluble organic UV-screening agents, chosen from liposoluble organic UVA-screening agents, liposoluble organic UVB-screening agents, and mixtures thereof.

The liposoluble organic UV-screening agents are in particular chosen from cinnamic derivatives; anthranilates; salicylic derivatives; dibenzoylmethane derivatives; camphor derivatives; benzophenone derivatives; β,β-diphenylacrylate derivatives; triazine derivatives; benzotriazole derivatives; benzalmalonate derivatives such as those mentioned in patent US 5 624 663; imidazolines p-aminobenzoic acid (PABA) derivatives; benzoxazole derivatives such as those described in patent applications EP0832642, EP1027883, EP1300137 and DE10162844; screening polymers and screening silicones such as those described in particular in application WO-93/04665; α-alkylstyrene -based dimers such as those described in patent application DE19855649; 4,4-diarylbutadienes such as those described in applications EP0967200, DE19746654, DE19755649, EP-A-1008586, EP1133980 and EP133981; merocyanine derivatives and merocyanines such as those described in patent US 4 195 999, application WO2004/006878, applications WO2008/090066, WO2011113718 and WO2009027258, and the documents IP COM JOURNAL N°000179675D published on 23 February 2009, IP COM JOURNAL N°000182396D published on 29 April 2009, IP COM JOURNAL N° 000189542D published on 12 November 2009, IP COM Journal N°IPCOM000011179D published on 04/03/2004; and mixtures thereof.

As examples of additional organic UV-screening agents, mention may be made of those denoted hereinbelow under their INCI name:
- dibenzoylmethane derivatives, such as butylmethoxydibenzoylmethane or avobenzone sold in particular under the trade name Parsol 1789 by the company DSM Nutritional Products;
- para-aminobenzoic acid derivatives, such as ethyl PABA, ethyl dihydroxypropyl PABA or ethylhexyl dimethyl PABA sold in particular under the name Escalol 507 by ISP;
- salicylic derivatives, such as homosalate sold in particular under the name Eusolex HMS by Rona/EM Industries, ethylhexyl salicylate sold in particular under the name Neo Heliopan OS by Symrise;
- cinnamic derivatives, such as ethylhexyl methoxycinnamate sold in particular under the trade name Parsol MCX by DSM Nutritional Products, isopropyl methoxycinnamate, isoamyl methoxycinnamate sold in particular under the trade name Neo Heliopan E 1000 by Symrise, cinoxate, diisopropyl methylcinnamate;
- β,β-diphenylacrylate derivatives, such as octocrylene sold in particular under the trade name Uvinul N539 by BASF, etocrylene, sold in particular under the trade name Uvinul N35 by BASF;
- benzophenone derivatives, such as benzophenone-1 sold in particular under the trade name Uvinul 400 by BASF, benzophenone-2 sold in particular under the trade name Uvinul D50 by BASF, benzophenone-3 or oxybenzone, sold in particular under the trade name Uvinul M40 by BASF, benzophenone-6 sold in particular under the trade name Helisorb 11 by Norquay, benzophenone-8 sold in particular under the trade name Spectra-Sorb UV-24 by American Cyanamid, benzophenone-12, n-hexyl 2-(4-diethylamino-2-hydroxybenzoyl)benzoate sold in particular under the trade name Uvinul A +, such as Uvinul A + Granular or in the form of a mixture with octylmethoxycinnamate in particular under the trade name Uvinul A + B by BASF;
- benzylidenecamphor derivatives, such as 3-benzylidenecamphor sold in particular under the name Mexoryl SD by Chimex, 4-methylbenzylidenecamphor sold in particular under the name Eusolex 6300 by Merck, polyacrylamidomethyl benzylidenecamphor sold in particular under the name Mexoryl SW by Chimex;
- phenyl benzotriazole derivatives, such as drometrizole trisiloxane sold in particular under the name Silatrizole by Rhodia Chimie;
- triazine derivatives, such as bis-ethylhexyloxyphenol methoxyphenyl triazine sold in particular under the trade name Tinosorb S by BASF, ethylhexyl triazone sold in particular under the trade name Uvinul T150 by BASF, diethylhexyl butamido triazone sold in particular under the trade name Uvasorb HEB by Sigma 3V, silicone triazines substituted with two aminobenzoate groups as described in patent EP0841341, in particular 2,4-bis-(n-butyl 4'-aminobenzalmalonate)-6-[(3-{1,3,3,3-tetramethyl-1-[(trimethylsilyloxy]disiloxanyl}propyl)amino]-s-triazine;
- anthranilic derivatives, such as menthyl anthranilate sold in particular under the trade name Neo Heliopan MA by Symrise;
- imidazoline derivatives, such as ethylhexyl dimethoxybenzylidene dioxoimidazoline propionate;
- benzalmalonate derivatives, such as dineopentyl 4'-methoxybenzalmalonate, polyorganosiloxane containing benzalmalonate functions, for instance Polysilicone-15, sold in particular under the trade name Parsol SLX by DSM;
- 4,4-diarylbutadiene derivatives, such as 1,1-dicarboxy(2,2'-dimethylpropyl)-4,4-diphenylbutadiene;
- benzoxazole derivatives, such as 2,4-bis[5-(1-dimethylpropyl)benzoxazol-2-yl-(4-phenyl)imino]-6-(2-ethylhexyl)imino-1,3,5-triazine, sold in particular under the name Uvasorb K2A by Sigma 3V;
- lipophilic merocyanine derivatives, such as octyl-5-N,N-diethylamino-2-phenysulfonyl-2,4-pentadienoate.

Preferably, the liposoluble organic screening agent(s) are chosen from butyl methoxy dibenzoylmethane, ethylhexylmethoxycinnamate, ethylhexyl salicylate, homosalate, butyl methoxydibenzoylmethane, octocrylene, benzophenone-3, n-hexyl 2-(4-diethylamino-2-hydroxybenzoyl)benzoate, 4-methylbenzylidenecamphor, bis-ethylhexyloxyphenol methoxyphenyl triazine, ethylhexyl triazone, diethylhexyl butamidotriazone, 2,4,6-tris(dineopentyl 4'-aminobenzalmalonate)-s-triazine, 2,4,6-tris-(diisobutyl 4'-aminobenzalmalonate)-s-triazine, 2,4-bis(dineopentyl 4'-aminobenzalmalonate)-6-(n-butyl 4 '-aminobenzoate)-s-triazine, drometrizole trisiloxane, polysilicone-15, 1,1-dicarboxy(2,2 '-dimethylpropyl)-4,4-diphenylbutadiene, 2,4-bis[5-1(dimethylpropyl)benzoxazol-2-yl(4-phenyl)imino]-6-(2-ethylhexyl)imino-1,3,5-triazine, and mixtures thereof.

More preferentially, the liposoluble organic screening agent(s) are chosen from homosalate, ethylhexyl salicylate, drometrizole trisiloxane, bis-ethylhexyloxyphenol methoxyphenyl triazine, octocrylene, butyl methoxydibenzoylmethane, ethylhexyl triazone, and mixtures thereof.

Among these liposoluble organic UV-screening agents, some of them are liquid at ambient temperature (20-25°C) under one atmosphere, whereas others are in a solid form (powder or granules) which can be dissolved in an oil.

The composition according to the present invention may comprise one or more insoluble organic UV-screening agents, chosen from insoluble organic UVA-screening agents, insoluble organic UVB-screening agents, and mixtures thereof.

The insoluble organic UV-screening agents according to the invention preferably have a mean particle size which ranges from 0.01 to 5 µm and more preferentially from 0.01 to 2 µm and more particularly from 0.020 to 2 µm.

The mean particle diameter is measured using a particle size distribution analyser of the Culter N4 PLUS type manufactured by Beckman Coulter Inc.

The insoluble organic screening agents according to the invention can be brought to the desired particulate form by any ad hoc means, such as in particular dry milling or milling in a solvent medium, sieving, atomization, micronization or pulverization.

The insoluble organic screening agents according to the invention in micronized form can in particular be obtained by means of a process of milling an insoluble organic UV-screening agent in the form of particles of coarse size in the presence of an appropriate surfactant making it possible to improve the dispersion of the resulting particles in the cosmetic formulations.

An example of a process for micronization of insoluble organic screening agents is described in applications GB-A-2 303 549 and EP-A-893119. The milling apparatus used according to these documents may be a jet, ball, vibration or hammer mill and preferably a high speed stirring mill or an impact mill and more particularly a rotating ball mill, a vibrating mill, a tube mill or a rod mill.

According to this particular process, use is made, as surfactants for milling said screening agents, of alkylpolyglucosides having the structure CₙH₂ₙ₊₁ O(C₆H₁₀O₅)ₓH in which n is an integer from 8 to 16 and x is the mean degree of polymerization of the unit (C₆H₁₀O₅) and ranges from 1.4 to 1.6. They may be chosen from C₁-C₁₂ esters of a compound having the structure CₙH₂ₙ₊₁O(C₆H₁₀O₅)ₓH and more particularly an ester obtained by reacting a C₁-C₁₂ carboxylic acid, such as formic, acetic, propionic, butyric, sulfosuccinic, citric or tartaric acid, with one or more free OH functions on the glucoside unit (C₆H₁₀O₅). Decylglucoside may in particular be mentioned as alkylpolyglucoside.

Said surfactants are generally used at a concentration ranging from 1% to 50% by weight and more preferentially from 5% to 40% by weight, relative to the insoluble screening agent in its micronized form.

The insoluble organic UV-screening agents in accordance with the invention may be chosen in particular from organic UV-screening agents of the oxalanilide type, of the triazine type, of the benzotriazole type; of the vinylamide type; of the cinnamide type; of the type comprising one or more groups which are benzazole and/or benzofuran, benzothiophene or of the indole type; of the aryl vinylene ketone type; of the phenylene bis-benzoxazinone derivative type; of the amide, sulfonamide or acrylonitrile carbamate derivative type, or mixtures thereof.

For the purpose for which it is used in the present invention, the term "benzazole" encompasses at the same time benzothiazoles, benzoxazoles and benzimidazoles.

Among the UV-screening agents of the oxalanilide type in accordance with the invention, mention may be made of those corresponding to formula (III) below: 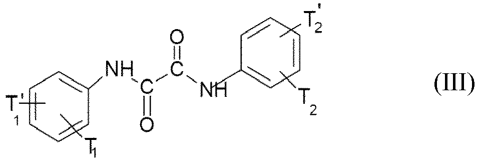 in which T₁, T'₁, T₂ and T'₂ denote, identically or differently, a C₁ to C₈ alkyl radical or a C₁ to C₈ alkoxy radical. These compounds are described in patent application WO 95/22959.

By way of examples, mention may be made of the commercial products Tinuvin 315® and Tinuvin 312® sold by the company BASF and respectively having the structure:

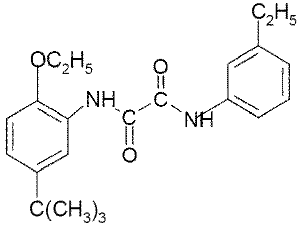

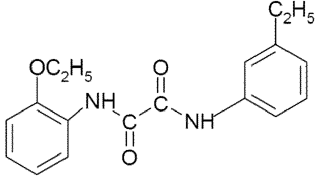

Among the insoluble UV-screening agents of the triazine type in accordance with the invention, mention may also be made of those corresponding to formula (IV) below: 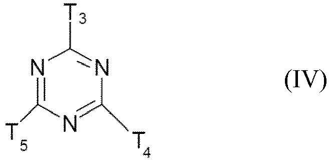 in which T₃, T₄ and T₅, independently, are phenyl, phenoxy or pyrrolo, in which the phenyl, phenoxy and pyrrolo are unsubstituted or substituted with one, two or three substituents chosen from OH, C₁-C₁₈ alkyl or C₁-C₁₈ alkoxy, C₁-C₁₈ carboxyalkyl, C₅-C₈ cycloalkyl, a methylbenzylidenecamphor group, and a -(CH=CH)ₙ(CO)-OT₆ group, with T₆ either C₁-C₁₈ alkyl or cinnamyl.

These compounds are described in WO 97/03642, GB 2286774, EP-743309, WO 98/22447 and GB 2319523.

Among the UV-screening agents of the triazine type in accordance with the invention, mention may also be made of insoluble derivatives of s-triazine bearing benzalmalonate and/or phenyl cyanoacrylate groups, such as those described in application EP-A-0790243 (which is an integral part of the content of the description).

Among these insoluble UV-screening agents of the triazine type, mention will more particularly be made of the following compounds:
- 2,4,6-tris(diethyl 4'-aminobenzalmalonate)-s-triazine,
- 2,4,6-tris(diisopropyl 4'-aminobenzalmalonate)-s-triazine,
- 2,4,6-tris(dimethyl 4'-aminobenzalmalonate)-s-triazine,
- 2,4,6-tris(ethyl α-cyano-4-aminocinnamate)-s-triazine.

Among the UV-screening agents of the triazine type in accordance with the invention, mention may also be made of insoluble derivatives of s-triazine bearing benzotriazole and/or benzothiazole groups, such as those described in application WO 98/25922 (which is an integral part of the content of the description).

Among these compounds, mention may more particularly be made of:
- 2,4,6-tris[(3'-benzotriazol-2-yl-2'-hydroxy-5'-methyl)phenylamino]-s-triazine,
- 2,4,6-tris[(3'-benzotriazol-2-yl-2'-hydroxy-5'-ter-octyl)phenylamino]-s-triazine.

Mention may also be made of the symmetrical triazines substituted with naphthalenyl groups or polyphenyl groups described in patent US 6 225 467, application WO2004/085412 (see compounds 6 and 9) or the document "Symmetrical Triazine Derivatives" IP.COM Journal, IP.COM INC West Henrietta, NY, US (20 September 2004), in particular 2,4,6-tris(di-phenyl)triazine and 2,4,6-tris(ter-phenyl)triazine which is reiterated in patent applications WO06/035000, WO06/034982, WO06/034991, WO06/035007, WO2006/034992, and WO2006/034985.

Among the insoluble organic UV-screening agents of the benzotriazole type in accordance with the invention, mention may be made of those of formula (V) below, as described in application WO 95/22959 (which is an integral part of the content of the description): 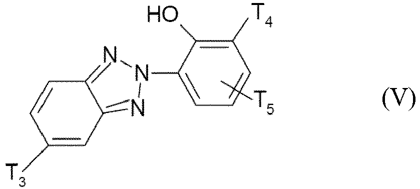 in which T₇ denotes a hydrogen atom or a C₁ to C₁₈ alkyl radical; and T₈ and T₉, which may be identical or different, denote a C₁ to C₁₈ alkyl radical optionally substituted with a phenyl.

By way of example of compounds of formula (V), mention may be made of the commercial products Tinuvin 328, 320, 234 and 350 from the company BASF, having the structure below:

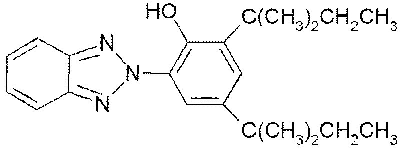

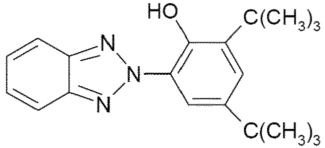

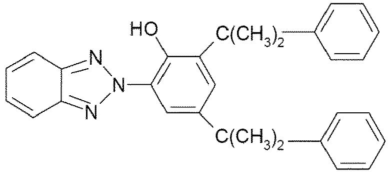

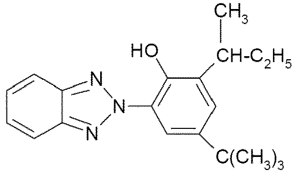

Among the insoluble organic UV-screening agents of the benzotriazole type in accordance with the invention, mention may be made of the compounds as described in patents US 5 687 521, US 5 373 037 and US 5 362 881, and in particular [2 ,4'-dihydroxy-3-(2H-benzotriazol-2-yl)-5-(1,1,3,3-tetramethylbutyl)-2'-n-octoxy-5'-benzoyl]diphenylmethane sold in particular under the name Mixxim PB30® by the company Fairmount Chemical, having the structure:

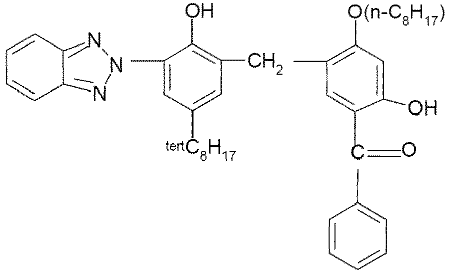

Among the insoluble organic UV-screening agents of the benzotriazole type in accordance with the invention, mention may be made of the methylenebis(hydroxyphenylbenzotriazole) derivatives having the structure below: 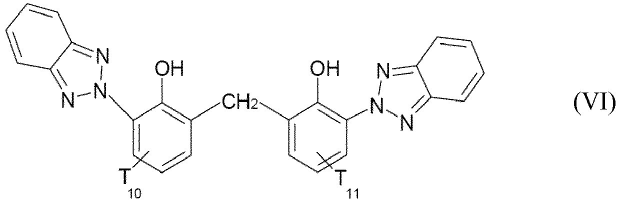 in which the T₁₀ and T₁₁ radicals, which may be identical or different, denote a C₁ to C₁₈ alkyl radical which may be substituted with one or more radicals chosen from a C₁ to C₄ alkyl radical, a C₅ to C₁₂ cycloalkyl radical or an aryl residue. These compounds are known per se and described in applications 5 US 5237 071, US 5 166 355, GB-A-2 303 549, DE 197 26 184 and EP-A-893 119 (which are an integral part of the description).

In formula (VI) defined above: the C₁ to C₁₈ alkyl groups may be linear or branched and are for example methyl, ethyl, n-propyl, isopropyl, n-butyl, isobutyl, tert-butyl, tert-octyl, n-amyl, n-hexyl, n-heptyl, n-octyl, isooctyl, n-nonyl, n-decyl, n-undecyl, n-dodecyl, tetradecyl, hexadecyl or octadecyl; the C₅ à C₁₂ cycloalkyl groups are for example cyclopentyl, cyclohexyle or cyclooctyl; the aryl groups are for example phenyl or benzyl.

Among the compounds of formula (VI), mention may be made of those having the structure below:

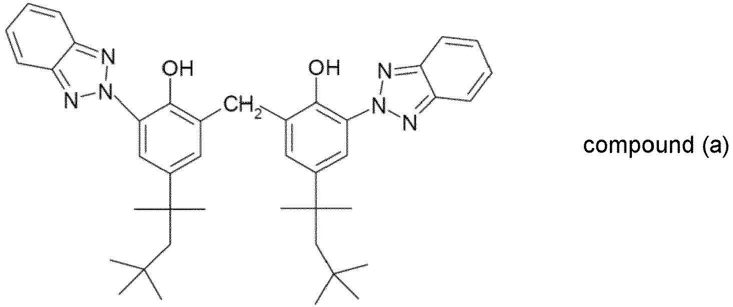

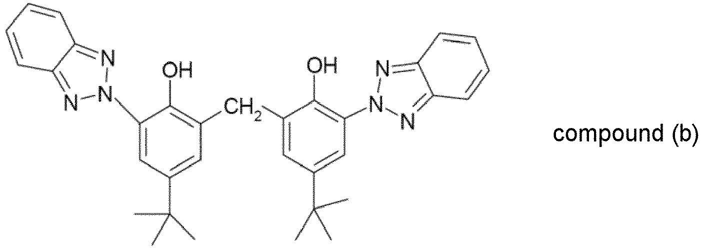

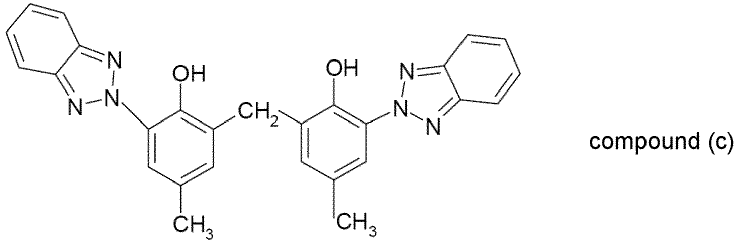

The compound (a) of nomenclature 2,2'-methylenebis[6-(2H-benzotriazol-2-yl)-4-(1,1,3,3-tetramethylbutyl)phenol] is in particular sold under the trade name Mixxim BB/200® by the company Fairmount Chemical.

The compound (c) of nomenclature 2,2'-methylenebis[6-(2H-benzotriazol-2-yl)-4-(methyl)phenol] is in particular sold in solid form under the trade name Mixxim BB/200® by the company Fairmount Chemical.

Among the insoluble organic screening agents of the vinylamide type, mention may be made for example of the compounds having the formula below which are described in application WO 95/22959 (which is an integral part of the content of the description):

T₁₂-(Y)ᵣ-C(=O)-C(T₁₃)=C(T₁₄)-N(T₁₅)(T₁₆) (VII)

in which T₁₂ is a C₁ to C₁₈, preferably C₁ to C₅, alkyl radical or a phenyl group which is optionally substituted with one, two or three radicals chosen from OH, C₁ to C₁₈ alkyl, C₁ to Cs alkoxy, or a -C(=O)-OT₁₇ group where T₁₇ is a C₁ to C₁₈ alkyl; T₁₃, T₁₄, T₁₅ and T₁₆, which may be identical or different, denote a C₁ to C₁₈, preferably C₁ to C₅, alkyl radical or a hydrogen atom; Y is N or O and r is 0 or 1.

Among these compounds, mention will more particularly be made of 4-octylamino-3-penten-2-one; ethyl-3-octylamino-2-butenoate; 3-octylamino-1-phenyl-2-buten-1-one and 3-dodecylamino-1-phenyl-2-buten-1-one.

Among the insoluble organic screening agents of the cinnamamide type in accordance with the invention, mention may also be made of the compounds as described in application WO 95/22959 (which is an integral part of the content of the description) and which correspond to the structure below: 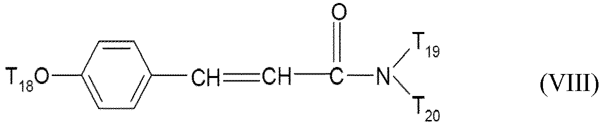 in which OT₁₈ is a hydroxyl or C₁ to C₄ alkoxy radical, preferably methoxy or ethoxy; T₁₉ is hydrogen or C₁ to C₄ alkyl, preferably methyl or ethyl; T₂₀ is a - (CONH)ₛ-phenyl group where s is 0 or 1 and the phenyl group may be substituted with one, two or three groups chosen from OH, C₁ to C₁₈ alkyl, C₁ to C₈ alkoxy, or a -C(=O)-OT₂₁ group where T₂₁ is a C₁ to C₁₈ alkyl and more preferentially T₂₁ is a phenyl, 4-methoxyphenyl or phenylaminocarbonyl group.

Mention may also be made of cinnamamide dimers such as those described in patent US 5 888 481, for instance the compound having the structure:

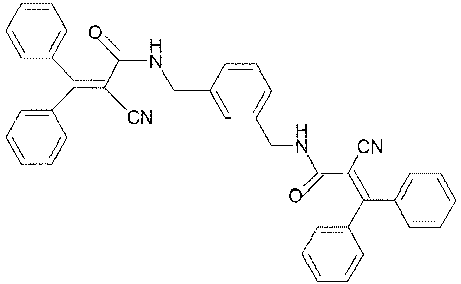

Among the insoluble organic screening agents of the benzazole type, mention may be made of those corresponding to one of the formulae below: 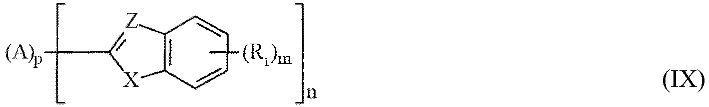 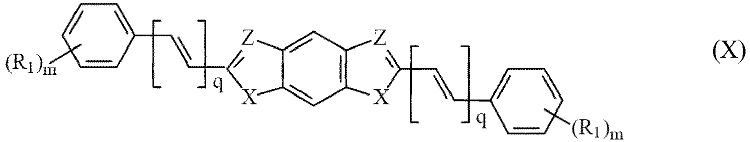 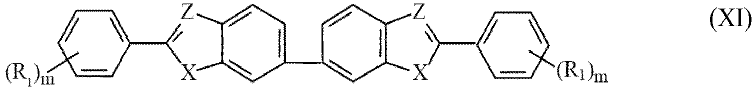 in which
- each of the symbols X independently represents an oxygen or sulfur atom or an NR₂ group, each of the symbols Z independently represents a nitrogen atom or a CH group,
- Each of the symbols R₁ independently represents an OH group, a halogen atom, a linear or branched C₁ to C₈ alkyl group, optionally containing a silicon atom, or a linear or branched C₁ to C₈ alkoxy group,
- each of the numbers m is independently 0, 1 or 2,
- n represents an integer ranging from 1 to 4,
- p is equal to 0 or 1,
- each of the numbers q is independently equal to 0 or 1,
- each of the symbols R₂ independently represents a hydrogen atom, or a benzyl or linear or branched C₁ to C₈ alkyl group, optionally containing a silicon atom,
- A represents a radical of valency n chosen from those of formulae: 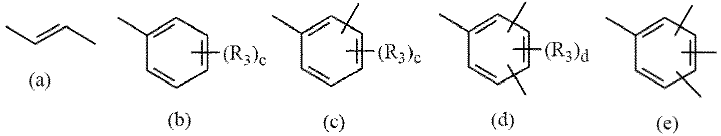 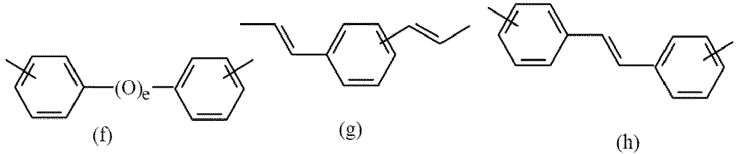 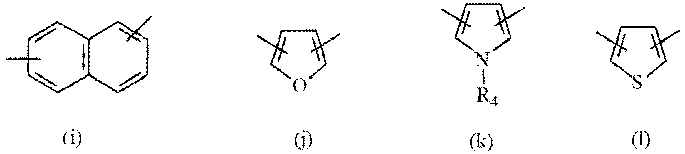 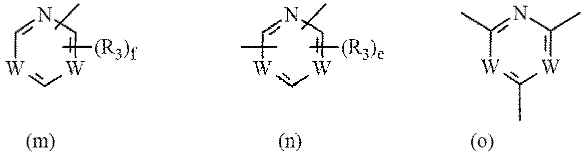
in which each of the symbols R₃ independently represents a halogen atom or a linear or branched C₁ to C₄ alkyl or alkoxy group or a hydroxyl group, and R₄ represents a hydrogen atom or a linear or branched C₁ to C₄ alkyl group, c = 0 - 4, d = 0 - 3, e = 0 or 1, and f = 0 - 2.

These compounds are in particular described in patents DE 676 103 and CH 350 763, patent US 5 501 850, patent US 5 961 960, patent application EP0669323, patent US 5 518 713, patent US 2 463 264, the article in J. Am. Chem. Soc., 79, 5706 - 5708, 1957, the article in J. Am. Chem. Soc., 82, 609 - 5 611, 1960, patent application EP0921126, and patent application EP712855.

By way of examples of preferred compounds of formula (IX) of the 2-arylbenzazole family, mention may be made of 2-benzoxazol-2-yl-4-methylphenol, 2-(1H-benzimidazol-2-yl)-4-methoxyphenol or 2-benzothiazol-2-ylphenol, these compounds possibly being prepared for example according to the processes described in patent CH 350 763.

By way of examples of preferred compounds of formula (IX) of the benzimidazolylbenzazole family, mention will be made of 2,2'-bis-benzimidazole, 5,5',6,6'-tetramethyl-2,2'-bis-benzimidazole, 5,5'-dimethyl-2,2'-bis-benzimidazole, 6-methoxy-2,2'-bis-benzimidazole, 2-(1H-benzimidazol-2-yl)benzothiazole, 2-(1H-benzimidazol-2-yl)benzoxazole and N,N'-dimethyl-2,2'-bis-benzimidazole, these compounds possibly being prepared according to the procedures described in patents US 5 961 960 and US 2 463 264.

By way of examples of preferred compounds of formula (IX) of the phenylenebenzazole family, mention will be made of 1,4-phenylene-bis-(2-benzoxazolyl), 1,4-phenylene-bis-(2-benzimidazolyl), 1,3-phenylene-bis-(2-benzoxazolyl), 1,2-phenylene-bis-(2-benzoxazolyl), 1,2-phenylene-bis-(benzimidazolyl), 1,4-phenylene-bis-(N-2-ethylhexyl-2-benzimidazolyl) and 1,4-phenylene-bis-(N-trimethylsilylmethyl-2-benzimidazolyl), these compounds possibly being prepared according to the procedures described in patent US 2 463 264 and in the publications J. Am. Chem. Soc., 82, 609 (1960) and J. Am. Chem. Soc., 79, 5706 -5708 (1957).

By way of examples of preferred compounds of formula (IX) of the benzofuranylbenzoxazole family, mention will be made of 2-(2-benzofuranyl)benzoxazole, 2-(benzofuranyl)-5-methylbenzoxazole and 2-(3-methyl-2-benzofuranyl)benzoxazole, these compounds possibly being prepared according to the procedures described in patent US 5 518 713.

As preferred compounds of formula (X), mention may for example be made of 2,6-diphenyl-1,7-dihydrobenzo[1,2-d;4,5-d']diimidazole corresponding to the formula: 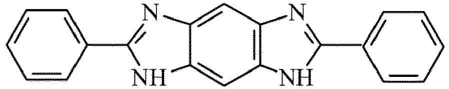 or 2,6-distyryl-1,7-dihydrobenzo[1,2-d; 4,5-d']diimidazole or else 2,6-di(p-tert-butylstyryl)-1,7-dihydrobenzo[1,2-d; 4,5-d']diimidazole, which can be prepared according to application EP 0 669 323.

As preferred compound of formula (XI), mention may be made of 5,5'-bis-[(phenyl-2)-benzimidazole] having the formula: 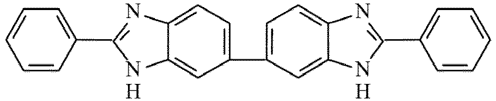 the preparation of which is described in J. Chim. Phys., 64, 1602 (1967).

Among these insoluble organic compounds which screen out UV radiation, preference is given most particularly to 2-(1H-benzimidazol-2-yl)benzoxazole, 6-methoxy-2,2'-bis-benzimidazole, 2-(1H-benzimidazol-2-yl)benzothiazole, 1,4-phenylenebis-(2-benzoxazolyl), 1,4-phenylene-bis-(2-benzimidazolyl), 1,3-phenylenebis-(2-benzoxazolyl), 1,2-phenylene-bis-(2-benzoxazolyl), 1,2-phenylenebis-(2-benzimidazolyl) and 1,4-phenylene-bis-(N-trimethylsilylmethyl-2-benzimidazolyl).

Among the insoluble organic screening agents of the aryl vinylene ketone type, mention may be made of those corresponding to one of formulae (XII) and (XIII) below: 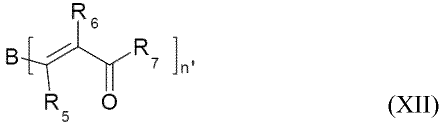 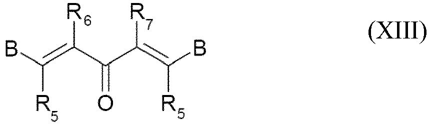 in which
- n' = 1 or 2,
- B, in formula (XII) when n'=1 or in formula (XIII), is an aryl radical chosen from formulae (a') to (d') below, or, in formula (XII) when n'=2, is a radical chosen from formulae (e') to (h') below: 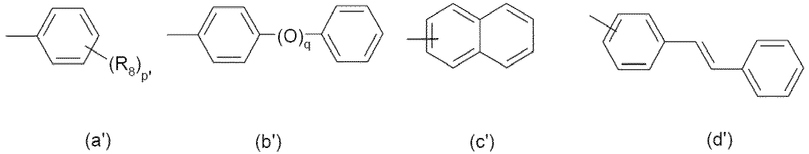 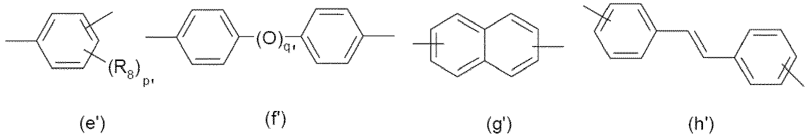 in which each of the symbols R₈ independently represents an OH group, a halogen atom, a linear or branched C₁ to C₆ alkyl group, optionally containing a silicon atom, a linear or branched C₁ to C₆ alkoxy group, optionally containing a silicon atom, a linear or branched C₁ to C₅ alkoxycarbonyl group, or a linear or branched C₁ to C₆ alkylsulfonamide group, optionally containing a silicon atom or an amino acid function,
- p' represents an integer ranging from 0 to 4,
- q' represents 0 or 1,
- R₅ represents hydrogen or an OH group,
- R₆ represents hydrogen, a linear or branched C₁ to C₆ alkyl group, optionally containing a silicon atom, a cyano group, a C₁ to C₆ alkylsulfonyl group, or a phenylsulfonyl group,
- R₇ represents a linear or branched C₁ to C₆ alkyl group, optionally containing a silicon atom, or a phenyl group which can form a bicycle and which is optionally substituted with one or two R₄ radicals,
- or R₆ and R₇ together form a monocyclic, bicyclic or tricyclic C₂ to C₁₀ hydrocarbon-based residue, optionally interrupted with one or more nitrogen, sulfur and oxygen atoms and possibly containing another carbonyl, and optionally substituted with a linear or branched C₁ to C₈ alkylsulfonamide group optionally containing a silicon atom or an amino acid function, on the condition that, when n'=1, R₆ and R₇ do not form a camphor nucleus.

By way of examples of insoluble compounds of formula (XII), in which n' = 1, which screen out UV radiation and which have a mean particle size of between 10 nm and 5 nm, mention may be made of the following families:
- compounds of the styryl ketone type as described in application JP 04 134 042, such as 1-(3,4-dimethoxyphenyl)-4,4-dimethyl-pent-1-en-3-one: 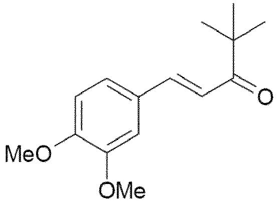
- compounds of the benzylidene cineole type such as those described in the article by E. Mariani et al, 16th IFSCC Congress, New York (1990), such as 1,3,3-trimethyl-5-(4-methoxybenzylidene)-2-oxabicyclo[2.2.2]octan-6-one: 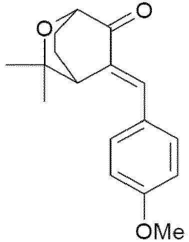
- compounds of the benzylidene chromanone type such as those described in application JP 04 134 043, for instance 3-(4-methoxybenzylidene)-2,3,4a,8a-tetrahydrochromen-4-one: 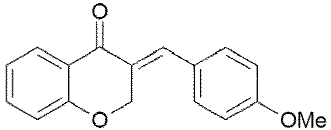
- compounds of the benzylidene thiochromanone type such as those described in application JP 04 134 043, for instance 3-(4-methoxybenzylidene)-2,3,4a,8a-tetrahydrochromen-4-thione: 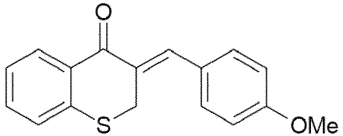
- compounds of the benzylidene quinuclidinone type such as those described in application EP 0 576 974, for instance 4-methoxybenzylidene-1-azabicyclo [2.2.2]octan-3 -one: 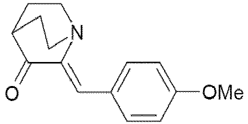
- compounds of the benzylidene cycloalcanone type such as those described in application FR 2 395 023, for instance 2-(4-methoxybenzylidene)cyclopentanone and 2-(4-methoxybenzylidene)cyclohexanone: 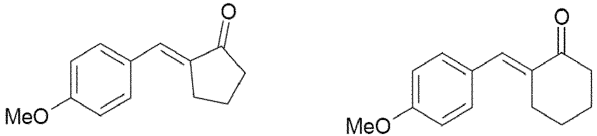
- compounds of the benzylidene hydantoin type such as those described in application JP 01 158 090, for instance 5-(3,4-dimethoxybenzylidene)imidazolidine-2,4-dione: 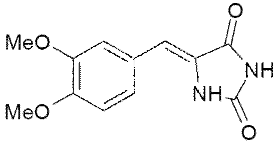
- compounds of the benzylidene indanone type such as those described in application JP 04 134 043, for instance 2-(4-methoxybenzylidene)indan-1-one: 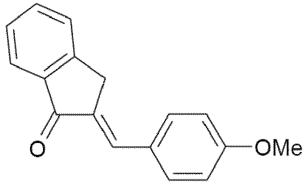
- compounds of the benzylidene tetralone type such as those described in application JP 04 134 043, for instance 2-(4-methoxybenzylidene)-3,4-dihydro-2H-naphthalen-1-one: 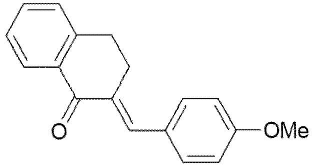
- compounds of the benzylidene furanone type such as those described in application EP 0 390 683, for instance 4-(4-methoxybenzylidene)-2,2,5,5-tetramethyldihydrofuran-3 -one: 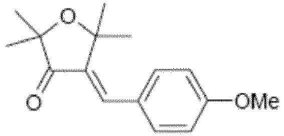
- compounds of the benzylidene benzofuranone type such as those described in application JP 04 134 041, for instance 2-benzlidenebenzofuran-3-one: 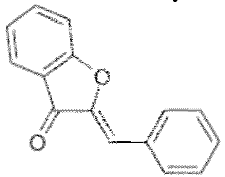
- compounds of the benzylidene indanedione type such as 2-(3,5-di(tert-butyl)-4-hydroxybenzylidene)indan-1,3-dione: 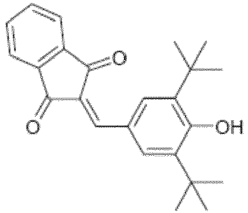
- compounds of the benzylidene benzothiofuranone type such as those described in application JP 04,134,043), for instance 2-benzylidenebenzo[b]thiophen-3-one: 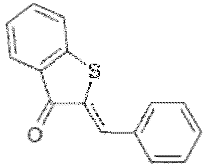
- compounds of the benzylidene barbiturate type such as 5-(4-methoxybenzylidene)-1,3-dimethylpyrimidine-2,4,6-trione: 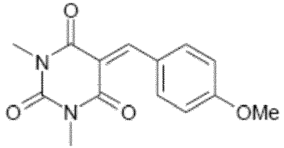
- compounds of the benzylidene pyrazolone type such as 4-(4-methoxybenzylidene)-5-methyl-2-phenyl-2,4-dihydropyrazol-3-one: 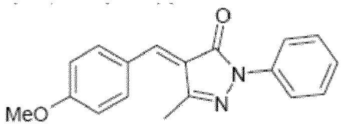
- compounds of the benzylidene imidazolone type such as 5-(4-methoxybenzylidene)-2-phenyl-3,5-dihydroimidazol-4-one: 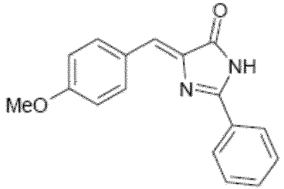
- compounds of the chalcone type such as 1-(2-hydroxy-4-methoxyphenyl)-3-phenylpropenone: 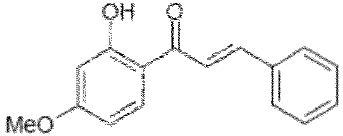
- benzylidene one compounds as described in document FR 2 506 156, for instance 3 -hydroxy-1 -(2-hydroxy-4-methoxyphenyl)-3 -phenylpropenone:

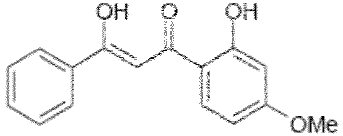

By way of examples of insoluble compounds of formula (X), in which n' = 2, which screen out UV radiation and which have a mean particle size of between 10 nm and 5 µm, mention may be made of the following families:
- compounds of the phenylene bis methylidene-nor-camphor type as described in document EP 0 693 471, for instance 1,4-phenylene-bis-{3-methylidenebicyclo[2.2.1]heptan-2-one}: 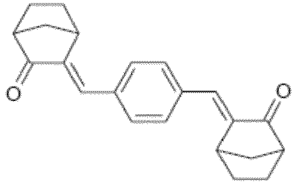
- compounds of the phenylene bis methylidene camphor type as described in document FR 2 528 420, for instance 1,4-phenylene-bis-{3-methylidene-1,7,7-trimethyl; bicyclo[2.2.1]heptan-2-one}: 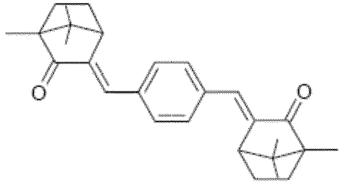 or 1,3-phenylene-bis-{3-methylidene-1,7,7-trimethylbicyclo [2.2.1]heptan-2-one} : 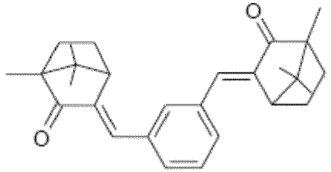
- compounds of the phenylene bis methylidene camphor sulfonamide type such as those described in document FR2 529 887, for instance ethyl or 2-ethylhexyl 1,4-phenylene-bis-3,3'-methylidenecamphor-10,10'-sulfonamide: 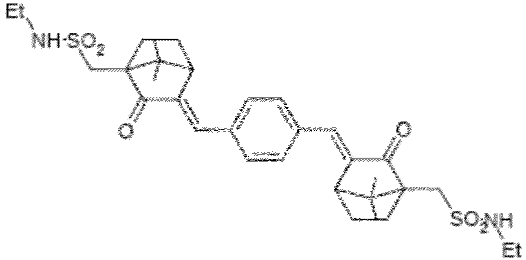 or 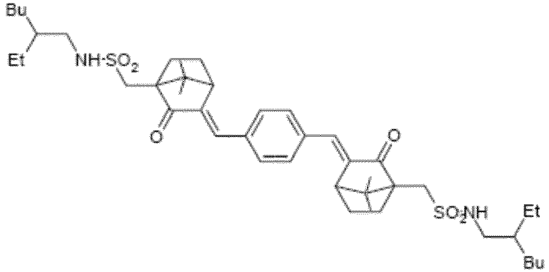
- compounds of the phenylene bis methylidene cineole type as described in the article E. Mariani et al, 16th IFSCC Congress, New York (1990), for instance 1,4-phenylene-bis-{5-methylidene-3,3-dimethyl-2-oxabicyclo[2.2.2]octan-6-one}: 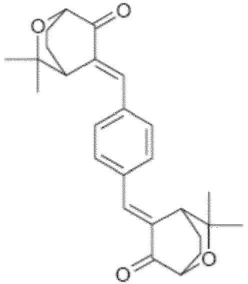
- compounds of the phenylene bis methylidene ketotricyclodecane type as described in application EP 0 694 521, for instance 1,4-phenylene-bis-(octahydro-4,7-methano-6-inden-5-one): 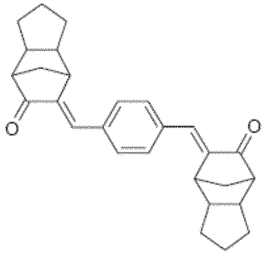
- compounds of the phenylene bis alkylene ketone type such as those described in application JP 04 134 041, for instance 1,4-phenylene-bis-(4,4-dimethylpent-1-en-3-one): 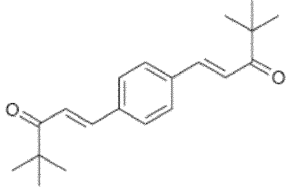
- compounds of the phenylene bis methylidene furanone type as described in application FR 2 638 354, for instance 1,4-phenylene-bis-(4-methylidene-2,2,5,5-tetramethyldihydrofuran-3 -one): 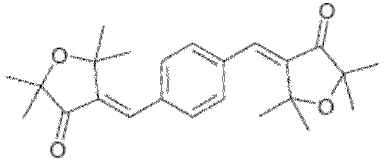
- compounds of the phenylene bis methylidene quinuclidinone type such as those described in application EP 0 714 880, for instance 1,4-phenylene-bis-{2-methylidene-1-azabicyclo[2.2.2]octan-3-one}:

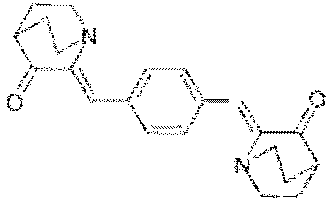

By way of compounds of formula (XIII), mention may be made of the following families:
- compounds of the bis benzylidene cycloalcanone type such as 2,5-dibenzylidenecyclopentanone: 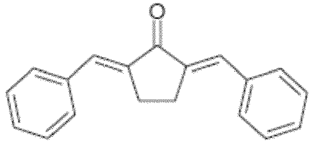
- compounds of the gamma pyrone type as described in document JP 04 290 882, for instance 2,6-bis-(3,4-dimethoxyphenyl)pyran-4-one:

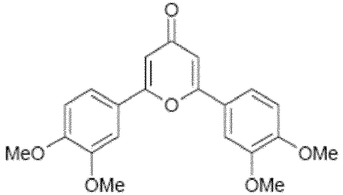

Among these insoluble organic compounds which filter out UV radiation, of the aryl vinyl ketone type, preference is given most particularly to the compounds of formula (XII) in which n'=2.

Among the insoluble organic screening agents of the phenylene bis-benzoxazinone type, mention may be made of those corresponding to formula (XIV) below: 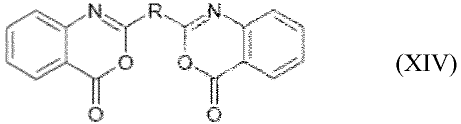 with R representing a divalent aromatic residue chosen from the formulae (e) to (h) below: 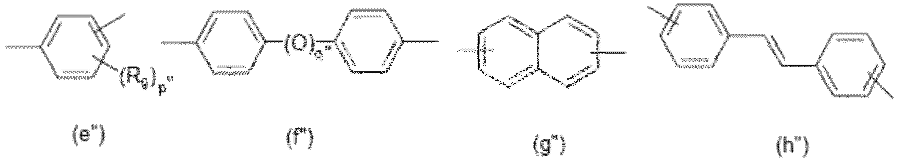 in which
- each of the symbols R₉ independently represents an OH group, a halogen atom, a linear or branched C₁ to C₆ alkyl group, optionally containing a silicon atom, a linear or branched C₁ to C₆ alkoxy group, optionally containing a silicon atom, a linear or branched C₁ to C₅ alkoxycarbonyl group, or a linear or branched C₁ to C₆ alkylsulfonamide group, optionally containing a silicon atom or an amino acid function,
- p" represents an integer ranging from 0 to 4,
- q" represents 0 or 1.

By way of examples of insoluble compounds of formula (XIV), which screen out UV radiation and which have a mean particle size of between 10 nm and 5 µm, mention may be made of the following derivatives:
- 2,2'-p-phenylenebis(3,1-benzoxazin-4-one), sold in particular under the trade name Cyasorb UV-3638® by the company Cytec,
- 2,2'-(4,4'-biphenylene)bis(3,1-benzoxazin-4-one),
- 2,2'-(2,6-naphthylene)bis(3,1-benzoxazin-4-one).

Among the insoluble organic screening agents of the amide, sulfonamide or acrylonitrile carbamate derivative type, mention may be made of those corresponding to formula (XV) below: 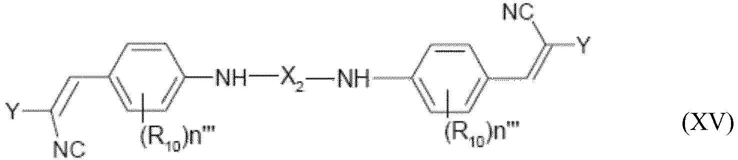 in which
- R₁₀ represents a linear or branched C₁ to C₈ alkyl group,
- n"' is 0, 1 or 2,
- X₂ represents a divalent radical of formula -(C=O)-R₁₁-(C=O)-, -SO₂-R₁₁-SO₂- or - (C=O)-O-R₁₁-O-(C=O)-,
- Y represents a -(C=O)-R₁₂ or -SO₂R₁₃ radical,
- R₁₁ represents a single bond or a linear or branched C₁ to C₃₀ alkylene or C₃ to C₃₀ alkenylene divalent radical which may bear one or more hydroxyl substituents and which may contain, in the carbon-based chain, one or more hetero atoms chosen from oxygen, nitrogen and silicon atoms,
- R₁₂ represents an -OR₁₄ or -NHR₁₄ radical,
- R₁₃ represents a linear or branched C₁ to C₃₀ alkyl radical, or a phenyl nucleus which is unsubstituted or substituted with C₁ to C₄ alkyl or alkoxy radicals,
- R₁₄ represents a linear or branched C₁ to C₃₀ alkyl or C₃ to C₃₀ alkenyl radical which may bear one or more hydroxyl substituents and which may contain, in the carbon-based chain, one or more hetero atoms chosen from oxygen, nitrogen and silicon atoms.

Although, in formula (XV) above, only the isomers in which the cyano substituent is in the cis position relative to the para-aminophenyl substituent are represented, this formula should be understood as also encompassing the corresponding trans isomers; for each of the two double bonds and independently, the cyano and para-aminophenyl substituents may be in the cis or trans configuration relative to one another.

By way of example, mention may be made of the dimer of 2-ethylhexyl 2-cyano-3-[4-(acetylamino)phenyl]acrylate of formula:

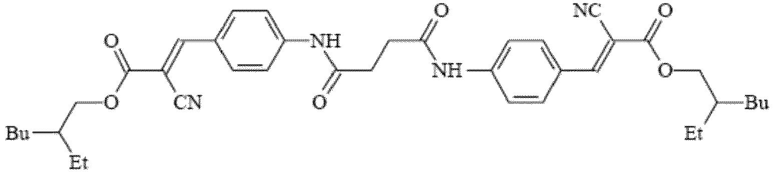

Another particular family of insoluble organic screening agents in accordance with the invention are the salts of polyvalent metals (for example Ca²⁺, Zn²⁺, Mg²⁺, Ba²⁺, Al³⁺ or Zr⁴⁺) of sulfonic or carboxylic organic screening agents such as the polyvalent metal salts of sulfonated derivatives of benzylidenecamphor, such as those described in application FR-A 2 639 347; the polyvalent metal salts of sulfonated derivatives of benzimidazole, such as those described in application EP-A-893119; the polyvalent metal salts of cinnamic acid derivatives, such as those described in application JP-87 166 517.

Mention may also be made of the metal, ammonium or substituted-ammonium complexes of UVA and/or UVB organic screening agents as described in patent applications WO93/10753, WO93/11095 and WO95/05150.

Among the insoluble organic UV-screening agents, mention may also be made of the compound 1,1'-(1,4-piperazinediyl)bis[1-[2-[4-(diethylamino)-2-hydroxybenzoyl]phenyl]methanone (CAS 919803-06-8) having the following structure: 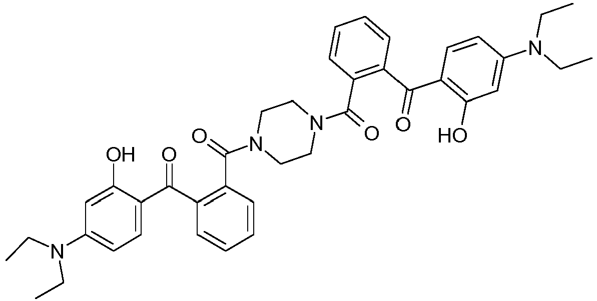 as described in patent application WO 2007/071 584; this compound advantageously being used in micronized form (mean size of 0.02 to 2 µm), which may be obtained, for example, according to the micronization process described in patent applications GB-A-2 303 549 and EP-A-893 119, and in particular in the form of an aqueous dispersion.

According to one particularly preferred form of the invention, use will be made of the insoluble organic UV-screening agents chosen from:
(i) symmetrical triazine screening agents substituted with naphthalenyl groups or polyphenyl groups described in patent US 6 225 467, patent application WO 2004/085 412 (see compounds 6 and 9) or the document "Symmetrical Triazine Derivatives", IP.COM IPCOM000031257 Journal, INC West Henrietta, NY, US (20 September 2004), in particular 2,4,6-tris(diphenyl)triazine and 2,4,6-tris(terphenyl)triazine, which is also mentioned in patent applications WO 06/035 000, WO 06/034 982, WO 06/034 991, WO 06/035 007, WO 2006/034 992 and WO 2006/034 985, these compounds advantageously being used in micronized form (mean particle size of 0.02 to 3 µm), which may be obtained, for example, according to the micronization process described in patent applications GB-A-2 303 549 and EP-A-893 119, and in particular in aqueous dispersion form;
(ii) the methylenebis(hydroxyphenylbenzotriazole) compounds of formula (XVI) below: 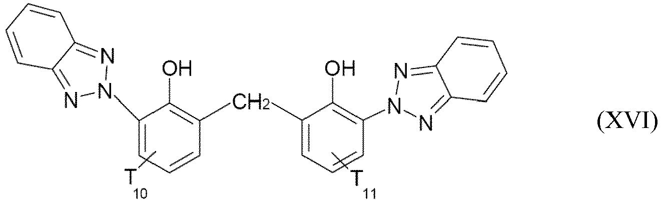 in which the T₁₀ and T₁₁ radicals, which may be identical or different, denote a C₁ to C₁₈ alkyl radical which may be substituted with one or more radicals chosen from a C₁ to C₄ alkyl radical, a C₅ to C₁₂ cycloalkyl radical or an aryl residue;
iii) and mixtures thereof.

According to one particularly preferred form of the invention, the methylenebis(hydroxyphenylbenzotriazole) compounds of formula (XVI) are in the form of an aqueous dispersion of particles having a mean particle size which ranges from 0.01 to 5 µm and more preferentially from 0.01 to 2 µm and more particularly from 0.020 to 2 µm with at least one surfactant of structure CₙH₂ₙ₊₁ O(C₆H₁₀O₅)ₓH in which n is an integer from 8 to 16 and x is the mean degree of polymerization of the unit (C₆H₁₀O₅) and ranges from 1.4 to 1.6. Said surfactant is preferably used at a concentration ranging from 1% to 50% by weight, and more preferentially from 5% to 40% by weight, relative to the benzotriazole screening agent, and the amount of benzotriazole screening agent of formula (I) in the aqueous dispersion preferably ranges from 10% to 50% by weight, and more preferentially from 30% to 50% by weight, relative to the total weight of the dispersion.

The mean particle diameter is measured using a particle size distribution analyser of the Coulter N4 PLUS® type manufactured by Beckman Coulter Inc.

According to one particularly preferred form of the invention, the methylenebis(hydroxyphenylbenzotriazole) compounds of formula (XVI) may be in the form of an aqueous dispersion of particles having a mean particle size which ranges from 0.02 to 2 µm and more preferentially from 0.01 to 1.5 µm and more particularly from 0.02 to 1 µm in the presence of at least one mono-(C₈-C₂₀) alkyl ester of poly glycerol having a degree of glycerol polymerization of at least 5, such as the aqueous dispersions described in application WO2009/063392.

As an example of surfactants which are mono-(C₈-C₂₀)alkyl esters of polyglycerol, mention may be made of decaglyceryl caprate, decaglyceryl laurate, decaglyceryl myristate, decaglyceryl oleate, decaglyceryl stearate, decaglyceryl isostearate, hexaglyceryl caprate, hexaglyceryl laurate, hexaglyceryl myristate, hexaglyceryl oleate, hexaglyceryl stearate, hexaglyceryl isostearate, pentaglyceryl caprate, pentaglyceryl laurate, pentaglyceryl myristate, pentaglyceryl oleate, pentaglyceryl stearate, and pentaglyceryl isostearate.

Use will more particularly be made of:
- decaglyceryl caprate such as the products sold under the following trade names: Sunsoft Q10Y®, Sunsoft Q10S®, Sunsoft Q12Y®, Sunsoft Q12S®, Sunsoft M12J® by the company Taiyo Kagaku Co. Ltd., Nikkol Decaglyn 1-L by the company Nikko Chemicals Co. Ltd, Ryoto-Polyglycerylester L-10D® and L-7D® by the company Mitsubishi-Kagaku Co. Ltd.,
- decaglyceryl laurate such as the products sold under the following trade names: Sunsoft Q14Y®, Sunsoft Q14S®, Sunsoft Q12Y®, Sunsoft Q12S®, Sunsoft M12J® by the company Taiyo Kagaku Co. Ltd., Nikkol Decaglyn 1-M® by the company Nikko Chemicals Co. Ltd, Ryoto-Polyglycerylester M-10D and M-7D by the company Mitsubishi-Kagaku Co. Ltd.,
- decaglyceryl stearate such as the products sold under the following trade names: Sunsoft Q18Y®, Sunsoft Q18S®, Sunsoft Q12Y®, Sunsoft Q12S®, Sunsoft M12J® by the company Taiyo Kagaku Co. Ltd., Nikkol Decaglyn 1-SV by the company Nikko Chemicals Co. Ltd, Ryoto-Polyglycerylester S-15D® by the company Mitsubishi-Kagaku Co. Ltd.,
- hexaglyceryl caprate such as the products sold under the following trade names: Nikkol Hexaglyn 1-L® by the company Nikko Chemicals Co. Ltd, Glysurf 6ML by the company Aoki Oil Industrial Co. Ltd., Unigly GL-106® by the company Nippon Oil & Fats Co. Ltd.,
- hexaglyceryl myristate such as the products sold under the following trade names: Nikkol Hexaglyn 1-M®, Nikkol Hexaglyn 1-OV® by the company Nikko Chemicals Co. Ltd, Glysurf 6ML® by the company Aoki Oil Industrial Co. Ltd., Unigly GL-106 by the company Nippon Oil & Fats Co. Ltd.,
- hexaglyceryl stearate such as the products sold under the following trade names: Nikkol Hexaglyn 1-M®, Nikkol Hexaglyn 1-SV® by the company Nikko Chemicals Co. Ltd, EmalexMSG-6K® by the company Nihon-Emulsion Co. Ltd., Unigly GL-106 by the company Nippon Oil & Fats Co. Ltd.,
- hexaglyceryl isostearate such as the products sold under the following trade names: Matsumate MI-610® by the company Matsumoto Fine Chemical Co. Ltd,
- pentaglyceryl caprate such as the products sold under the following trade names: Sunsoft A10E®, by the company Taiyo Kagaku Co. Ltd.,
- pentaglyceryl laurate such as the products sold under the following trade names: Sunsoft A12E®, Sunsoft A121E® by the company Taiyo Kagaku Co. Ltd.,
- pentaglyceryl myristate such as the products sold under the following trade names: Sunsoft A14E®, Sunsoft A141E® by the company Taiyo Kagaku Co. Ltd.,
- pentaglyceryl oleate such as the products sold under the following trade names: Sunsoft A17E®, Sunsoft A171E® by the company Taiyo Kagaku Co. Ltd.,
- pentaglyceryl stearate such as the products sold under the following trade names: Sunsoft A18E®, Sunsoft A181E® by the company Taiyo Kagaku Co. Ltd.

Among these surfactants, those having an HLB greater than or equal to 14.5, and more preferentially greater than or equal to 15, are preferably used. As examples of surfactants which are mono-(C₈-C₂₀)alkyl esters of polyglycerol having a degree of glycerol polymerization of at least 5 and having an HLB greater than or equal to 14.5, mention may be made of decaglyceryl caprate, decaglyceryl laurate, decaglyceryl myristate, decaglyceryl oleate, decaglyceryl stearate, decaglyceryl isostearate, hexaglyceryl laurate, pentaglyceryl caprate, pentaglyceryl laurate, pentaglyceryl myristate, pentaglyceryl oleate, and pentaglyceryl stearate. As examples of surfactants which are mono-(C₈-C₂₀)alkyl esters of polyglycerol having a degree of glycerol polymerization of at least 5 and having an HLB greater than or equal to 15, mention may be made of decaglyceryl caprate and decaglyceryl laurate.

The amount of methylenebis(hydroxyphenylbenzotriazole) compound of formula (XVI) in the aqueous dispersion preferably ranges from 10% to 50% by weight, and more preferentially from 30% to 50% by weight, relative to the total weight of the dispersion.

Preferentially, the weight ratio between the amount of the methylenebis(hydroxyphenylbenzotriazole) compound and the amount of the mono-(C₈-C₂₀)alkyl ester of polyglycerol ranges from 0.05 to 0.5, and preferentially from 0.1 to 0.3.

In these aqueous dispersions, use will preferentially be made, as methylenebis(hydroxyphenylbenzotriazole) compound of formula (XVI), of the compound 2,2'-methylenebis[6-(2H-benzotriazol-2-yl)-4-(1,1,3,3-tetramethylbutyl)phenol] having the structure: 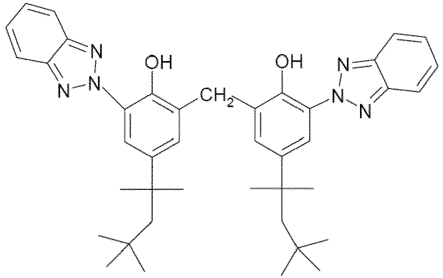 such as the commercial product sold under the name Tinosorb M® by BASF which is an aqueous dispersion comprising decylglucoside, xanthan gum and propylene glycol (INCI name: Methylene Bis-Benzotriazolyl Tetramethylbutylphenol (and) Aqua (and) Decyl Glucoside (and) Propylene Glycol (and) Xanthan Gum).

Preferably, the insoluble organic UV-screening agent is methylene bis-benzotriazolyl tetramethylbutylphenol.

Preferably, the composition according to the present invention comprises one or more liposoluble organic UV-screening agents, more preferentially chosen from dibenzoylmethane compounds, salicylic compounds, β,β-diphenylacrylate compounds, benzophenone compounds, phenyl benzotriazole compounds, triazine compounds and mixtures thereof, and even better still from homosalate, ethylhexyl salicylate, drometrizole trisiloxane, bis-ethylhexyloxyphenol methoxyphenyl triazine, octocrylene, butyl methoxydibenzoylmethane, ethylhexyl triazone, n-hexyl 2-(4-diethylamino-2-hydroxybenzoyl)benzoate, diethylhexyl butamido triazone and mixtures thereof.

The amount of UV-screening agent(s) present in the composition according to the invention may range from 0.1% to 50% by weight, relative to the total weight of the composition. It preferably ranges from 10% to 50% by weight, more preferentially from 20% to 50% by weight and better still ranges from 20% to 40% by weight, relative to the total weight of the composition.

The amount of UV-screening agent(s) present in the oily phase according to the invention may range from 50% to 100% by weight, relative to the total weight of the oily phase. It preferably ranges from 70% to 99% by weight, more preferentially from 80% to 98% by weight and even better still from 90% to 97% by weight, relative to the total weight of the oily phase.

### Anionic crosslinked hydrophilic polymers

The composition according to the present invention also comprises one or more anionic crosslinked hydrophilic polymers.

For the purposes of the present invention, the term "hydrophilic polymer" is intended to mean a polymer which, when introduced into water at a concentration equal to 1% by weight, at 25°C, gives a macroscopically homogeneous solution of which light transmittance, at a wavelength equal to 500 nm, through a sample 1 cm thick, is at least 10%, which corresponds to an absorbance [abs = -log(transmittance)] of less than 1.5.

The anionic crosslinked hydrophilic polymer(s) according to the invention do not generally contain monomers comprising fatty chains, i.e. they do not comprise a repeat unit having a CₙH₂ₙ₊₁ alkyl group wherein the integer n is greater than or equal to 6, preferably greater than or equal to 7.

For the purposes of the present invention, the term "crosslinked" is intended to mean a polymer in which the monomer has been polymerized with an olefinically polyunsaturated monomer commonly used for crosslinking polymers obtained by radical polymerization.

As crosslinking agents, mention may be particularly made of divinylbenzene, diallyl ether, dipropylene glycol diallyl ether, polyglycol diallyl ethers, triethylene glycol divinyl ether, hydroquinone diallyl ether, ethylene glycol or tetraethylene glycol di(meth)acrylate, trimethylolpropane triacrylate, methylenebisacrylamide, methylenebismethacrylamide, triallylamine, triallyl cyanurate, diallyl maleate, tetraallylethylenediamine, tetraallyloxyethane, trimethylolpropane diallyl ether, allyl (meth)acrylate, allylic ethers of alcohols of the sugar series, or other allyl or vinyl ethers of polyfunctional alcohols, and also the allylic esters of phosphoric and/or vinylphosphonic acid derivatives, or mixtures of these compounds.

According to one preferred embodiment of the invention, the crosslinking agent is chosen from methylenebisacrylamide, allyl methacrylate and trimethylolpropane triacrylate (TMPTA), and mixtures thereof. The degree of crosslinking generally ranges from 0.01 mol% to 10 mol% and more particularly from 0.2 mol% to 2 mol% relative to the polymer.

The anionic crosslinked hydrophilic polymer(s) used in the composition of the present invention can advantageously be chosen from crosslinked 2-acrylamido-2-methylpropanesulfonic acid homo- and copolymers, homo- and copolymers of at least one α,β-ethylenically unsaturated carboxylic acid monomer, and mixtures thereof. These polymers can optionally be totally or partially neutralized.

The anionic crosslinked hydrophilic polymers used in the composition of the present invention can thus be crosslinked homopolymers or copolymers comprising at least one 2-acrylamidomethylpropanesulfonic acid (AMPS®) monomer, in a form partially or totally neutralized with a mineral base such as sodium hydroxide or potassium hydroxide.

They are preferably totally or almost totally neutralized, i.e. at least 90% neutralized.

The AMPS® polymers that are suitable for use in the invention are water-soluble or water-dispersible. In this case, they are:
- either "homopolymers" comprising only AMPS® monomers and one or more crosslinking agents such as those defined above;
- or copolymers obtained from one or more AMPS® monomers, from one or more hydrophilic or hydrophobic ethylenically unsaturated monomers and from one or more crosslinking agents such as those defined above. The hydrophobic ethylenically unsaturated monomers do not comprise a fatty chain and are preferably present in small amounts.

For the purposes of the present invention, the term "fatty chain" is intended to mean a hydrocarbon-based chain comprising at least 6 carbon atoms.

The crosslinked hydrophilic AMPS® copolymers according to the present invention contain one or more co-monomers which may be chosen in particular from water-soluble ethylenically unsaturated monomers, hydrophobic monomers, and mixtures thereof.

The water-soluble comonomers may be ionic or non-ionic.

Among the ionic water-soluble comonomers, examples that may be mentioned include the following compounds, and salts thereof:
- (meth)acrylic acid,
- styrenesulfonic acid,
- vinylsulfonic acid and (meth)allylsulfonic acid,
- vinylphosphonic acid,
- maleic acid,
- itaconic acid,
- crotonic acid,
- water-soluble vinyl monomers of formula (A) below: 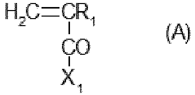 in which
   - R₁ is chosen from H, -CH₃, -C₂H₅ and -C₃H₇,
   - X₁ is chosen from alkyl oxides of type -OR₂ where R₂ is a linear or branched, saturated or unsaturated hydrocarbon-based radical containing from 1 to 6 carbon atoms, substituted with at least one sulfonic (-SO₃-) and/or sulfate (-SO₄-) and/or phosphate (-PO₄H₂-) group.

Among the non-ionic water-soluble comonomers, examples that may be mentioned include:
- (meth)acrylamide,
- N-vinylacetamide and N-methyl-N-vinylacetamide,
- N-vinylformamide and N-methyl-N-vinylformamide,
- maleic anhydride,
- vinylamine,
- N-vinyllactams comprising a cyclic alkyl group containing from 4 to 9 carbon atoms, such as N-vinylpyrrolidone, N-butyrolactam and N-vinylcaprolactam,
- vinyl alcohol of formula CH₂=CHOH,
- water-soluble vinyl monomers of formula (B) below: 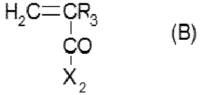 in which
   - R₃ is chosen from H, -CH₃, -C₂H₅ and -C₃H₇,
   - X₂ is chosen from alkyl oxides of the type -OR₄ where R₄ is a linear or branched, saturated or unsaturated hydrocarbon-based radical containing from 1 to 6 carbon atoms, optionally substituted with a halogen (iodine, bromine, chlorine or fluorine) atom; a hydroxyl (-OH) group; ether.

Mention is made, for example, of glycidyl (meth)acrylate, hydroxyethyl methacrylate, and (meth)acrylates of ethylene glycol, of diethylene glycol or of polyalkylene glycol.

Among the hydrophobic co-monomers without a fatty chain, mention may be made, for example, of:
- styrene and derivatives thereof, such as 4-butylstyrene, α-methylstyrene and vinyltoluene;
- vinyl acetate of formula CH₂=CH-OCOCH₃;
- vinyl ethers of formula CH₂=CHOR in which R is a linear or branched, saturated or unsaturated hydrocarbon-based radical containing from 1 to 6 carbons;
- acrylonitrile;
- caprolactone;
- vinyl chloride and vinylidene chloride;
- silicone derivatives, which, after polymerization, result in silicone polymers such as methacryloxypropyltris(trimethylsiloxy)silane and silicone methacrylamides;
- hydrophobic vinyl monomers of formula (C) below: 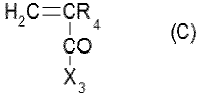 in which
   R₄ is chosen from H, -CH₃, -C₂H₅ and -C₃H₇;
   X₃ is chosen from alkyl oxides of the type -OR₅ where R₅ is a linear or branched, saturated or unsaturated hydrocarbon-based radical containing from 1 to 6 carbon atoms.

Mention is made, for example, of methyl methacrylate, ethyl methacrylate, n-butyl (meth)acrylate, tert-butyl (meth)acrylate, cyclohexyl acrylate and isobutyl (meth)acrylate.

The water-soluble or water-dispersible AMPS® polymers of the invention preferably have a molar mass ranging from 50 000 g/mol to 10 000 000 g/mol, preferably from 80 000 g/mol to 8 000 000 g/mol, and even more preferably from 100 000 g/mol to 7 000 000 g/mol.

As crosslinked hydrophilic homopolymers in accordance with the present invention, mention may for example be made of:
- crosslinked sodium acrylamido-2-methylpropanesulfonate polymers, such as that used in the commercial product Simulgel 800 (CTFA name: Sodium Polyacryloyldimethyl Taurate), crosslinked ammonium acrylamido-2-methylpropanesulfonate polymers (INCI name: Ammonium Polydimethyltauramide) such as those described in patent EP 0 815 928 B1 and such as the product sold under the trade name Hostacerin AMPS® by the company Clariant.
- crosslinked acrylamide/sodium acrylamido-2-methylpropanesulfonate copolymers, such as that used in the commercial product Sepigel 305 (INCI name: Polyacrylamide/C₁₃-C₁₄ Isoparaffin/ Laureth-7) or that used in the commercial product sold under the name Simulgel 600 (INCI name: Acrylamide/Sodium acryloyldimethyltaurate/Isohexadecane/Polysorbate-80) by the company SEPPIC;
- copolymers of AMPS® and of vinylpyrrolidone or vinylformamide, such as that used in the commercial product sold under the name Aristoflex AVC® by the company Clariant (CTFA name: Ammonium Acryloyldimethyltaurate/VP copolymer) but neutralized with sodium hydroxide or potassium hydroxide;

- copolymers of AMPS® and of sodium acrylate, for instance the AMPS/sodium acrylate copolymer, such as that used in the commercial product sold under the name Simulgel EG® by the company SEPPIC or under the trade name Sepinov EM (CTFA name: Hydroxyethyl acrylate/Sodium acryloyldimethyltaurate copolymer);
- copolymers of AMPS® and of hydroxyethyl acrylate, for instance the AMPS®/hydroxyethyl acrylate copolymer, such as that used in the commercial product sold under the name Simulgel NS® by the company SEPPIC (CTFA name: Hydroxyethyl acrylate/Sodium acryloyldimethyltaurate copolymer (and) Squalane (and) Polysorbate 60), or such as the product sold under the name Sodium acrylamido-2-methylpropanesulfonate/Hydroxyethyl acrylate copolymer, such as the commercial product Sepinov EMT 10 (INCI name: Hydroxyethyl acrylate/Sodium acryloyldimethyltaurate copolymer).

As preferred crosslinked hydrophilic AMPS® copolymers in accordance with the present invention, mention may be made of copolymers of AMPS® and of hydroxyethyl acrylate.

The anionic crosslinked hydrophilic polymers may also be homopolymers or copolymers of at least one α,β-ethylenically unsaturated carboxylic acid monomer. They may in particular be chosen from copolymers derived from the polymerization of at least one monomer (a) chosen from α,β-ethylenically unsaturated carboxylic acids or esters thereof, with at least one ethylenically unsaturated monomer (b).

For the purposes of the present invention, the term "copolymers " is intended to mean both copolymers obtained from two sorts of monomers and those obtained from more than two sorts of monomers, such as terpolymers obtained from three sorts of monomers.

Preferably, these copolymers are chosen from copolymers derived from the polymerization:
- of at least one monomer of formula (1) below: 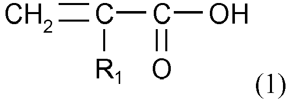 in which R₁ denotes H or CH₃ or C₂H₅, i.e. acrylic acid, methacrylic acid or ethacrylic acid monomers.

Among the polymers containing at least one carboxylic acid monomer, mention may also be made of sodium polyacrylates such as those sold under the name Cosmedia SP®, or Cosmedia SPL® as an inverse emulsion containing about 60% solids, an oil (hydrogenated polydecene) and a surfactant (PPG-5 Laureth-5), both sold by the company Cognis.

Mention may also be made of partially neutralized sodium polyacrylates that are in the form of an inverse emulsion comprising at least one polar oil, for example the product sold under the name Luvigel® EM by the company BASF.

These polymers may also be chosen from crosslinked (meth)acrylic acid homopolymers.

Examples that may be mentioned include the products sold by Lubrizol under the names Carbopol 910, 934, 940, 941, 934 P, 980, 981, 2984, 5984 and Carbopol Ultrez 10 Polymer, or by 3V-Sigma under the name Synthalen® K, Synthalen® L or Synthalen® M.

Preferably, the anionic crosslinked hydrophilic polymer(s) present in the composition according to the invention are chosen from polymers comprising at least one α,β-ethylenically unsaturated carboxylic acid monomer, more preferentially from crosslinked (meth)acrylic acid homopolymers, and even better still from Carbopol 980 (CTFA name: carbomer), Carbopol Ultrez 10 Polymer, and mixtures thereof.

The amount of the anionic crosslinked hydrophilic polymer(s) present in the composition of the invention preferably ranges from 0.05% to 5% by weight, more preferentially from 0.08% to 2.5% by weight, and even better still from 0.1% to 1.5% by weight, relative to the total weight of the composition.

### Surfactants

The composition according to the present invention also comprises one or more surfactants having an HLB less than or equal to 5.

The term "HLB" is well known to those skilled in the art, and denotes the hydrophilic-lipophilic balance of a surfactant at 25°C in the Griffin sense.

The term "hydrophilic-lipophilic balance (HLB)" is intended to mean the equilibrium between the size and the strength of the hydrophilic group and the size and the strength of the lipophilic group of the surfactant. The HLB value according to Griffin is defined in J. Soc. Cosm. Chem. 1954 (volume 5), pages 249-256.

The surfactant(s) having an HLB less than or equal to 5 may be ionic or non-ionic. Use may in particular be made of the surfactants having an HLB less than or equal to 5 which are mentioned in the reference handbook McCutcheons Emulsifiers & Dertergents, international edition from 1998 et seq.

Reference may also be made to Kirk-Othmer's Encyclopedia of Chemical Technology, volume 22, pp. 333-432, 3rd edition, 1979, Wiley, for the definition of the emulsifying properties and functions of surfactants, in particular pp. 347-377 of this reference, for non-ionic surfactants.

Preferably, the surfactants of the invention have an HLB less than or equal to 4.

More particularly, the surfactants suitable for the present invention are non-ionic.

The non-ionic surfactants having an HLB less than or equal to 5 may in particular be chosen from alkyl and polyalkyl esters of poly(ethylene oxide); oxyalkylenated fatty alcohols; alkyl and polyalkyl ethers of poly(ethylene oxide); polyoxyethylenated or non-polyoxyethylenated alkyl and polyalkyl esters of sorbitan; non-polyoxyethylenated alkyl and polyalkyl ethers of sorbitan; alkyl- and polyalkylglycosides or polyglycosides, in particular alkyl- and polyalkylglucosides or polyglucosides; alkyl and polyalkyl esters of sucrose; polyoxyethylenated or non-polyoxyethylenated alkyl and polyalkyl esters of glycerol; polyoxyethylenated or non-polyoxyethylenated alkyl and polyalkyl ethers of glycerol; and mixtures thereof, these compounds comprising at least one fatty chain comprising from 6 to 30 carbon atoms, preferably from 12 to 30 carbon atoms, and more preferentially from 14 to 24 carbon atoms.

They may be chosen from fatty alcohols, fatty α-diols and (C₁-₂₀)alkylphenols, these compounds being polyethoxylated and/or polypropoxylated and/or polyglycerolated, the number of ethylene oxide and/or propylene oxide groups possibly ranging from 1 to 15, preferably from 1 to 10, and the number of glycerol groups possibly ranging from 1 to 10, preferably from 2 to 6; these compounds comprising at least one fatty chain comprising from 6 to 30 carbon atoms, preferably from 12 to 30 carbon atoms, and more preferentially from 14 to 24 carbon atoms.

Mention may also be made of condensates of ethylene oxide and of propylene oxide with fatty alcohols; polyethoxylated fatty amides preferably having from 2 to 30 ethylene oxide units, polyglycerolated fatty amides comprising on average from 1 to 5, and in particular from 1.5 to 4, glycerol groups; fatty acid esters of sorbitan, fatty acid esters of sucrose, polyoxyalkylenated and preferably polyoxyethylenated fatty acid esters containing from 2 to 10 mol of ethylene oxide, including oxyethylenated plant oils.

Alkyl and polyalkyl esters of poly(ethylene oxide) that are preferably used include those with a number of ethylene oxide (EO) units ranging from 2 to 10, and more preferentially from 2 to 6. Mention may, for example, be made of stearate 2 EO, diethylene glycol monostearate, and polyoxyethylenated monostearate comprising 5 EO.

As oxyalkylenated fatty alcohols, in particular oxyethylenated and/or oxypropylenated alcohols, use is made of those preferably having from 1 to 10 and more preferentially from 2 to 5 oxyethylene and/or oxypropylene units, in particular ethoxylated C₈-C₂₄ and preferably C₁₂-C₁₈ fatty alcohols such as stearyl alcohol ethoxylated with 2 oxyethylene units (CTFA name: Steareth-2) such as Brij 72 sold by the company Uniqema. Mention may also be made of cetyl ether 2 EO, phytosterol 2 EO, steareth 4, beheneth 2 and oleth-2.

As non-polyoxyethylenated alkyl and polyalkyl esters of sorbitan, use is preferably made of sorbitan monostearate and sorbitan tristearate.

Polyoxyethylenated or non-polyoxyethylenated alkyl and polyalkyl ethers of sorbitan that are preferably used include those with a number of ethylene oxide (EO) units ranging from 0 to 10 and more preferentially from 0 to 5.

Alkyl and polyalkyl glucosides or polyglucosides that are preferably used include those containing an alkyl group comprising from 12 to 30 carbon atoms and preferably from 16 to 22 carbon atoms, and containing a glucoside group preferably comprising from 1 to 5 and in particular 1, 2 or 3 glucoside units. Among the alkylpolyglucosides that can be used, mention may be made in particular of arachidylglucoside.

Examples of alkyl and polyalkyl esters of sucrose that may be mentioned are Crodesta F150, sucrose monolaurate sold under the name Crodesta SL 40, and the products sold by Ryoto Sugar Ester, for instance sucrose palmitate sold under the reference Ryoto Sugar Ester P1670, Ryoto Sugar Ester LWA1695 or Ryoto Sugar Ester 01570 or sucrose distearate.

Polyoxyethylenated or non-polyoxyethylenated alkyl and polyalkyl esters of glycerol that are preferably used include those with a number of ethylene oxide (EO) units ranging from 0 to 10 and a number of glycerol units ranging from 1 to 8. More preferentially, the alkyl and polyalkyl esters of glycerol are non- polyoxyethylenated.

Mention may, for example, be made of glyceryl monooleate, hexaglyceryl monolaurate, diglyceryl distearate, glyceryl stearate, glyceryl isostearate, tetraglyceryl tristearate, decaglyceryl decastearate, diglyceryl monostearate, hexaglyceryl tristearate, decaglyceryl pentastearate, glyceryl monobehenate, glyceryl dibehenate and the ester of glycerol and of palmitic and stearic acids.

Polyoxyethylenated or non-polyoxyethylenated alkyl and polyalkyl ethers of glycerol that are preferably used include those with a number of ethylene oxide (EO) units ranging from 0 to 10 and more preferentially from 0 to 5, and a number of glycerol units ranging from 1 to 10.

Preferably, the surfactant(s) having an HLB less than or equal to 5 present in the composition according to the invention are chosen from steareth-2, beheneth-2, oleth-2, sucrose distearate, glyceryl monooleate, glyceryl stearate, glyceryl isostearate, diglyceryl distearate, tetraglyceryl tristearate, decaglyceryl decastearate, diglyceryl monostearate, hexaglyceryl tristearate, decaglyceryl pentastearate, sorbitan monostearate, sorbitan tristearate, diethylene glycol monostearate, the ester of glycerol and of palmitic and stearic acids, glyceryl monobehenate, glyceryl dibehenate, and mixtures thereof; and more preferentially glyceryl monooleate.

The amount of the surfactant(s) having an HLB less than or equal to 5 present in the composition according to the invention preferably ranges from 0.1% to 3% by weight, more preferentially from 0.2% to 2% by weight, and even better still from 0.3% to 1% by weight, relative to the total weight of the composition.

### Silicone copolymers

The composition according to the present invention also comprises one or more silicone copolymers resulting from the copolymerization of at least one monomer of the type of an ester of a carboxylic acid and of an alcohol comprising a C₆ to C₃₀ fatty chain, with at least one monomer containing a polyalkylsiloxane chain.

Among the monomers of the type ester of carboxylic acid and of alcohol comprising a fatty chain, that can be used according to the present invention, mention may in particular be made of C₆ to C₃₀, preferably C₇ to C₂₄, fatty-chain alkyl acrylates, methacrylates, maleates, fumarates, itaconates and crotonates, and mixtures thereof.

Preferably, the monomer(s) of the type ester of carboxylic acid and of alcohol comprising a C₆ to C₃₀ fatty chain are chosen from esters of (meth)acrylic acid and of alcohol comprising a C₆ to C₃₀, preferably C₇ to C₂₄, fatty chain.

In other words, the monomer(s) of the type ester of a carboxylic acid and of alkyl comprising a C₆ to C₃₀ fatty chain are preferentially chosen from the compounds of structure (XVII) and mixtures thereof 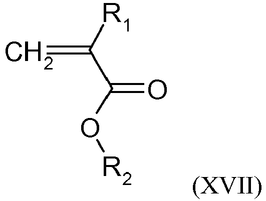 in which
R₁ represents a hydrogen atom or a methyl, and
R₂ represents a linear or branched, saturated or unsaturated C₆ to C₃₀, preferably C₇ to C₂₄, alkyl chain.

R₂ advantageously represents a branched, saturated or unsaturated C₇ to C₂₄ alkyl chain.

According to a first particularly preferred embodiment, R₁ represents a hydrogen atom and R₂ represents a branched, saturated or unsaturated C₇ to C₁₂ alkyl chain, more preferentially a 2-ethylhexyl group.

According to a second particularly preferred embodiment, R₁ represents a hydrogen atom and R₂ represents a linear, saturated or unsaturated C₁₄ to C₂₀ alkyl chain, more preferentially a stearyl group.

Preferably, the monomer(s) containing a polyalkylsiloxane chain are chosen from monomers of the type (meth)acrylic acid esterified with a silicone group of structure (XVIII) 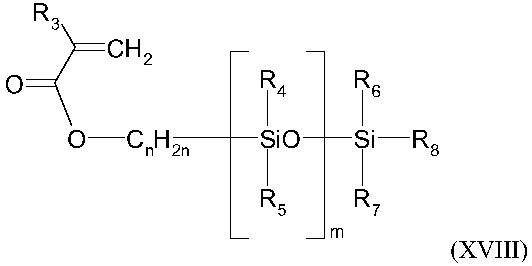 in which
R₃ represents a hydrogen atom or a methyl, and
R₄, R₅, R₆, R₇ and R₈, which may be identical or different, represent, independently of one another, a linear or branched, saturated or unsaturated C₁ to C₃₀ alkyl chain, n and m represent, independently of each other, an integer greater than or equal to 1.

Preferably, R₃ represents a methyl.

Preferably, R₄, R₅, R₆, R₇ and R₈, which may be identical or different, represent, independently of one another, a linear or branched, saturated or unsaturated C₁ to C₂₄, and more preferentially C₁ to C₁₂, alkyl chain.

According to one particularly preferred embodiment, R₃, R₄, R₅, R₆, R₇ and R₈ represent a methyl.

The silicone copolymer(s) according to the present invention may optionally also comprise one or more additional monomers, and in particular one or more monomers which are an ester of a carboxylic acid and of an alcohol of C₁ to C₅. Preferably, the additional monomer(s) are chosen from esters of (meth)acrylic acid and of a C₁ to C₄ alcohol and mixtures thereof, more preferentially from methyl esters of (meth)acrylic acid, butyl esters of (meth)acrylic acid, ethyl esters of (meth)acrylic acid and mixtures thereof.

Preferably, the silicone copolymer(s) present in the composition according to the invention are chosen from copolymers resulting from the copolymerization of at least one monomer of structure (XVII) in which R₁ represents a hydrogen atom and R₂ represents a branched, saturated or unsaturated, C₇ to C₂₄ alkyl chain, with at least one monomer of structure (XVIII) in which R₃, R₄, R₅, R₆, R₇ and R₈ all represent a methyl group, n is an integer between 1 and 6, and m represents an integer greater than or equal to 1; and mixtures thereof.

The silicone copolymer(s) are generally obtained according to the usual methods of polymerization and grafting, for example by radical polymerization of at least one monomer of polydimethylsiloxane type comprising at least one polymerizable radical group (polymerizable for example on one of the ends of the chain or at both ends), as previously described, and of at least one monomer of the type ester of carboxylic acid and of alkyl comprising a C₇ to C₃₀ fatty chain, as described, for example, in documents US-A-5 061 481 and US-A-5 219 560.

The silicone copolymer(s) obtained generally have a molecular weight ranging from about 3000 to 200 000 and preferably from about 5000 to 100 000.

The silicone copolymer(s) used in the composition according to the present invention may be in their native form or in a form dispersed in a solvent such as lower alcohols comprising from 2 to 8 carbon atoms, for instance isopropyl alcohol, or oils, for instance silicone oils, preferably volatile silicone oils, such as cyclopentasiloxane or a dimethicone, or else hydrocarbon-based oils, such as liquid paraffins or liquid isoparaffins, such as isododecane.

Preferably, the solvent is chosen from oils, and more preferentially from cyclopentasiloxane, linear pentadimethylsiloxane, and mixtures thereof.

The silicone copolymer(s) that can be used in the composition according to the present invention are preferentially chosen from copolymers of stearyl methacrylate/methyl methacrylate/propyl methacrylate containing a polydimethylsiloxane chain; copolymers of methyl methacrylate/butyl methacrylate/2-ethylhexyl acrylate/propyl methacrylate containing a polydimethylsiloxane chain; and mixtures thereof.

In particular, mention may be made of the acrylate/ dimethicone copolymers (INCI name) sold by the company Shin-Etsu under the trade names KP-561 in which the copolymer of stearyl methacrylate, of methyl methacrylate, and of propyl methacrylate comprising a polydimethylsiloxane group); KP-541 in which the copolymer is dispersed in isopropyl alcohol; KP-545 in which the copolymer of methyl methacrylate, of butyl methacrylate, of 2-ethylhexyl acrylate and of propyl methacrylate comprising a polydimethylsiloxane group is dispersed in decamethylcyclopentasiloxane; KP-545L in which the copolymer of methyl methacrylate, of butyl methacrylate, of 2-ethylhexyl acrylate and of propyl methacrylate comprising a polydimethylsiloxane group is dispersed in linear pentadimethylsiloxane; and KP-550 in which the copolymer of methyl methacrylate, of butyl methacrylate, of 2-ethylhexyl acrylate and of propyl methacrylate comprising a polydimethylsiloxane group is dispersed in isododecane.

Preferably, the silicone copolymer(s) present in the composition according to the invention are chosen from the products sold under the trade names KP-545 and KP-545L by the company Shin-Etsu.

The amount of the silicone copolymer(s) present in the composition of the invention preferably ranges from 0.5% to 10% by weight, more preferentially from 1% to 8% by weight, and even better still from 2% to 5% by weight, relative to the total weight of the composition.

The weight ratio between the amount of silicone copolymer(s) and the amount of anionic crosslinked hydrophilic polymer(s) present in the composition according to the invention is less than or equal to 5, preferably less than or equal to 4.5, and more preferentially ranges from 1 to 4.5.

### Efficiency boosters

The composition according to the present invention may optionally also comprise one or more efficiency boosters.

For the purposes of the present application, the term "efficiency booster" is intended to mean a raw material which does not itself screen out UV radiation, but which makes it possible to amplify the screening performance of the composition of the invention.

By way of examples of efficiency boosters, mention may be made of particles of copolymer of styrene and of acrylates, such as those sold under the name Sunspheres Powder by Dow Chemicals; waxes such as polymethylene wax (Cirebelle 303 sold by Cirebelle); gelling lipophilic polymers such as the semicrystalline polyacrylates sold under the names Interlimer IPA 13-1, Interlimer IPA 13-6 or Uniclear 100 VG; and mixtures thereof.

According to one preferred embodiment, the composition according to the invention also comprises one or more particles of copolymer of styrene and of acrylates, sold under the name Sunspheres Powder by Dow Chemicals.

### Insoluble particles

The composition according to the present invention may optionally also comprise one or more insoluble particles, not having any UV-screening property or efficiency booster property.

For the purposes of the present application, the term "insoluble particle" is intended to mean a particle having a solubility in water of less than 0.1% by weight and a solubility of less than 0.1% by weight in the majority of organic solvents such as liquid paraffin, fatty alcohol benzoates and fatty acid triglycerides, for example Miglyol 812® sold by the company Dynamit Nobel. This solubility, determined at 25°C, is defined as the amount of product in solution in the solvent at equilibrium with an excess of solid in suspension.

These particles make it possible to obtain excellent sensory or optical properties after application of the composition to the skin.

These particles may be chosen from nylon, such as Orgasol 2002 sold by Arkema; polymethylsilsesquioxane, for example the methylsilsesquioxane resin microbeads sold under the name Tospearl 145A by the company Momentive Performance Materials; poly(methyl methacrylate), for instance the hollow PMMA spheres sold under the name Covabeads LH 85 by the company Sensient; starches such as corn starch; talc; silica; crosslinked polyacrylate microspheres such as Aquakeep 10 SH-NFC sold by the company Sumitomo Seika; perlite such as Optimat 2550 OR sold by the company World Minerals; iron oxide pigments or titanium oxide pigments and mixtures thereof.

Preferably, the composition according to the invention also comprises nylon particles, such as Orgasol 2002.

### Additives

The composition according to the present invention may optionally also comprise one or more additives, different from the compounds of the invention and normally used in cosmetics, and particularly in the field of anti-sun products, care products and makeup products.

The aqueous phase may optionally comprise surfactants different from the surfactants having an HLB less than or equal to 5, such as those mentioned above; alcohols such as ethanol; glycols, such as dipropylene glycol and butylene glycol; glycerol; active agents; salts; organic particles; amphiphilic polymers, such as the Pemulens TR1 or TR2 or Carbopol ETD2020, sold by the company Lubrizol; hydrophilic polymers, such as poly(N-vinylpyrrolidone); polysaccharides, for instance guar gums, xanthan gums and cellulose-based derivatives; water-soluble or water-dispersible silicone derivatives, for instance acrylic silicones, polyether silicones and cationic silicones; and mixtures thereof.

As active agents, mention may in particular be made of vitamins (A, C, E, K, PP, etc), alone or as a mixture, and also derivatives thereof; keratolytic and/or desquamating agents (salicylic acid and derivatives thereof, alpha-hydroxy acids, ascorbic acid and derivatives thereof); anti-inflammatories; calmatives; depigmenting agents; tensioning agents such as synthetic polymers; plant proteins; polysaccharides of plant origin optionally in the form of microgels; wax dispersions; mixed silicates and colloidal particles of inorganic fillers; matting agents; agents for preventing hair loss and/or hair restorers; or anti-wrinkle agents; and mixtures thereof.

The oily phase may optionally also comprise lipophilic gelling agents; surfactants different from the surfactants having an HLB less than or equal to 5, such as those mentioned above; waxes; organic or mineral particles; oils of alkane, silicone and fluoro type; and mixtures thereof.

Needless to say, those skilled in the art will take care to select this or these optional additional compound(s) such that the advantageous properties intrinsically associated with the composition according to the invention are not, or are not substantially, adversely affected by the envisaged addition(s).

The above adjuvants may generally be present in an amount, for each of them, of between 0 and 20% by weight relative to the total weight of the composition.

### The emulsion

The composition according to the invention is in the form of an oil-in-water emulsion (also known as direct emulsion, or abbreviated to O/W emulsion), containing droplets of oil dispersed in a continuous aqueous phase.

As indicated previously, the compositions according to the invention comprise at least one continuous aqueous phase.

The aqueous phase contains water and optionally other water-soluble or water-miscible organic solvents.

An aqueous phase that is suitable for use in the invention may comprise, for example, a water chosen from a natural spring water, such as water from La Roche-Posay, water from Vittel or waters from Vichy, or a floral water.

The water-soluble or water-miscible solvents that are suitable for use in the invention comprise short-chain monoalcohols, for example C₂-C₄ monoalcohols, such as ethanol or isopropanol; diols or polyols, such as ethylene glycol, propylene glycol, 1,3-butylene glycol, hexylene glycol, diethylene glycol, dipropylene glycol, propane-1,3-diol, pentylene glycol, caprylyl glycol, 2-ethoxyethanol, diethylene glycol monomethyl ether, triethylene glycol monomethyl ether, glycerol and sorbitol, and mixtures thereof.

According to a preferred embodiment, use may more particularly be made of ethanol, propylene glycol, glycerol, propane-1,3-diol, and mixtures thereof.

The aqueous phase of the composition according to the invention comprises at least water in a total quantity greater than or equal to 30% by weight, preferably greater than or equal to 50% by weight, and better still greater than or equal to 60% by weight, relative to the total weight of the aqueous phase.

In particular, the water may be present in a quantity ranging from 30 to 99% by weight, preferably from 50 to 99% by weight, and better still from 60 to 98% by weight, relative to the total weight of the aqueous phase.

The water may be present in a total quantity greater than or equal to 30% by weight, preferably greater than or equal to 45% by weight, and preferentially ranges from 45% to 65% by weight, relative to the total weight of the composition.

As indicated above, the composition according to the invention comprises at least one oily phase dispersed in the aqueous phase.

For the purposes of the invention, the term "oily phase" is intended to mean a phase comprising at least one oil and all of the liposoluble and lipophilic ingredients and the fatty substances used for the formulation of the compositions of the invention.

The term "oil" is intended to mean any fatty substance that is in liquid form at ambient temperature (25°C) and at atmospheric pressure. (1.013 × 10⁵ Pa).

The term "fatty substance" is intended to mean an organic compound that is insoluble in water at ambient temperature (25°C) and at atmospheric pressure (1.013 × 10⁵ Pa) (solubility of less than 5% by weight, and preferably less than 1% by weight, even more preferably less than 0.1% by weight). They bear in their structure at least one hydrocarbon-based chain including at least 6 carbon atoms and/or a sequence of at least two siloxane groups. In addition, the fatty substances are generally soluble in organic solvents under the same temperature and pressure conditions, for instance chloroform, dichloromethane, carbon tetrachloride, ethanol, benzene, toluene, tetrahydrofuran (THF), liquid petroleum jelly or decamethylcyclopentasiloxane.

The oils suitable for the invention may be volatile or non-volatile.

The oils suitable for the invention may be chosen from hydrocarbon-based oils, silicone oils and fluoro oils, and mixtures thereof.

A hydrocarbon-based oil that is suitable for use in the invention may be an animal hydrocarbon-based oil, a plant hydrocarbon-based oil, a mineral hydrocarbon-based oil or a synthetic hydrocarbon-based oil.

An oil that is suitable for use in the invention may be advantageously chosen from mineral hydrocarbon-based oils, plant hydrocarbon-based oils, synthetic hydrocarbon-based oils and silicone oils, and mixtures thereof.

For the purposes of the present invention, the term "silicone oil" is intended to mean an oil comprising at least one silicon atom, and in particular at least one Si-O group.

The term "hydrocarbon-based oil" is intended to mean an oil containing mainly hydrogen and carbon atoms.

The term "fluoro oil" is intended to mean an oil comprising at least one fluorine atom.

A hydrocarbon-based oil that is suitable for use in the invention may also optionally comprise oxygen, nitrogen, sulfur and/or phosphorus atoms, for example in the form of hydroxyl, amine, amide, ester, ether or acid groups, and in particular in the form of hydroxyl, ester, ether or acid groups.

The oily phase may comprise one or more volatile or non-volatile hydrocarbon-based oils and/or one or more volatile and/or non-volatile silicone oils.

For the purposes of the invention, the term "volatile oil" is intended to mean an oil that is capable of evaporating on contact with the skin or the keratin fibre in less than one hour, at ambient temperature and atmospheric pressure. The volatile oil(s) of the invention are volatile cosmetic oils that are liquid at ambient temperature with a non-zero vapour pressure, at ambient temperature and atmospheric pressure ranging in particular from 0.13 Pa to 40 000 Pa (10⁻³ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" is intended to mean an oil that remains on the skin or the keratin fibre at ambient temperature and atmospheric pressure for at least several hours, and that in particular has a vapour pressure of less than 10⁻³ mmHg (0.13 Pa).

Among the non-volatile hydrocarbon-based oils that can be used according to the invention, mention may be made of glyceride triesters and in particular caprylic/capric acid triglycerides such as those sold by the company Stearineries Dubois or those sold under the names Miglyol 810®, 812® and 818® by the company Dynamit Nobel, fatty amides such as isopropyl N-lauroyl sarcosinate, such as the product sold under the trade name Eldew SL 205® from Ajinomoto, synthetic esters, and in particular isononyl isononanoate, diisopropyl sebacate, C₁₂-C₁₅ alkyl benzoate, such as the product sold under the trade name Finsolv TN® or Witconol TN® by the company Witco or Tegosoft TN® by the company Evonik Goldschmidt, 2-ethylphenylbenzoate, such as the commercial product sold under the name X-Tend 226® by the company ISP, and fatty alcohols, in particular octyldodecanol.

As volatile hydrocarbon-based oils that may be used according to the invention, mention may be made in particular of hydrocarbon-based oils having from 8 to 16 carbon atoms and in particular of branched C₈-C₁₆ alkanes, such as C₈-C₁₆ isoalkanes of petroleum origin (also known as isoparaffins), such as isododecane (also known as 2,2,4,4,6-pentamethylheptane), isodecane or isohexadecane, the oils sold under the Isopar or Permethyl trade names, branched C₈-C₁₆ esters, isohexyl neopentanoate, and mixtures thereof.

Among the non-volatile silicone oils, mention may be made of non-volatile polydimethylsiloxanes (PDMSs), polydimethylsiloxanes comprising alkyl or alkoxy groups which are pendent and/or at the end of the silicone chain, which groups each contain from 2 to 24 carbon atoms, or phenyl silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates.

Among the volatile silicone oils, mention may for example be made of volatile linear or cyclic silicone oils, in particular those with a viscosity at 25°C of less than or equal to 8 centistokes (8×10⁻⁶ m²/s) and in particular containing from 2 to 7 silicon atoms, these silicones optionally comprising alkyl or alkoxy groups containing from 1 to 10 carbon atoms. As volatile silicone oils that may be used in the invention, mention may be made in particular of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane and mixtures thereof.

Among the volatile fluoro oils, mention may be made of nonafluoromethoxybutane, decafluoropentane, tetradecafluorohexane and dodecafluoropentane, and mixtures thereof.

An oily phase according to the invention may also comprise other fatty substances, mixed with or dissolved in the oil.

Another fatty substance that may be present in the oily phase may be, for example:
- a fatty acid, for example chosen from fatty acids comprising from 8 to 30 carbon atoms, such as lauric acid, palmitic acid, oleic acid and stearic acid;
- a wax chosen from waxes such as lanolin, beeswax, carnauba or candelilla wax, paraffin waxes, lignite waxes, microcrystalline waxes, ceresin or ozokerite, or synthetic waxes, such as polyethylene waxes or Fischer-Tropsch waxes;
- a gum chosen from silicone gums (dimethiconol);
- a pasty compound, such as polymeric or non-polymeric silicone compounds, esters of a glycerol oligomer, arachidyl propionate, fatty acid triglycerides and derivatives thereof;
- and mixtures thereof.

Preferably, the oil(s) present in the composition according to the invention are chosen from silicon oils, and more preferentially from cyclopentasiloxane, linear pentadimethylsiloxane and mixtures thereof.

The amount of aqueous phase preferably ranges from 50% to 95% by weight, more preferentially from 50% to 90% by weight, better still from 50% to 80% by weight and even more preferentially from 55% to 75% by weight, relative to the total weight of the composition.

The amount of oily phase preferably ranges from 5% to 50% by weight and more preferentially from 10% to 45% by weight, relative to the total weight of the composition.

The numerical mean size of the droplets of oil, present in the composition of the invention, preferably ranges from 0.4 to 20 microns and more preferentially from 5 to 15 microns.

This numerical mean size can be measured using a particle size analyser.

The emulsifying processes that can be used to prepare the compositions according to the invention may, for example, be of paddle or propeller and rotor-stator type.

The examples that follow serve to illustrate the invention without, however, being limiting in nature.

### EXAMPLES

In the examples that follow, all the amounts are given as weight percentages of active material (AM) relative to the total weight of the composition.

### EXAMPLE 1

### 1) Preparation of the compositions

The composition (A1) according to the present invention, and the comparative compositions (B1), (B2) and (B3) which follow were prepared from ingredients of which the contents are indicated in the table below.

| | Invention A1 | Comparative B1 | Comparative B2 | Comparative B3 |
|---|---|---|---|---|
| Triethanolamine | 0.25 | 0.25 | 0.25 | 0.25 |
| Phenoxyethanol | 0.7 | 0.7 | 0.7 | 0.7 |
| Disodium EDTA | 0.05 | 0.05 | 0.05 | 0.05 |
| Styrene/acrylate copolymer | 0.9 | 0.9 | 0.9 | 0.9 |
| Carbomer^{(*)} | 0.3 | 0.3 | 0.3 | - |
| Crosslinked C₁₀-₃₀ alkyl acrylate polymer | - | - | - | 0.3 |
| Propanediol | 3 | 3 | 3 | 3 |
| Homosalate | 10 | 10 | 10 | 10 |
| Ethylhexyl salicylate | 5 | 5 | 5 | 5 |
| Drometrizone trisiloxane | 0.9 | 0.9 | 0.9 | 0.9 |
| Bis(ethylhexylphenol)methoxyphenyltriazine | 1.7 | 1.7 | 1.7 | 1.7 |
| Octocrylene | 5 | 5 | 5 | 5 |
| Butyl methoxy dibenzoylmethane | 5 | 5 | 5 | 5 |
| Ethylhexyl triazone | 2.35 | 2.35 | 2.35 | 2.35 |
| Glyceryl monooleate | 0.4 | - | 0.4 | 0.4 |
| Polysorbate 20 | - | 0.4 | - | - |
| Nylon 12 | 0.8 | 0.8 | 0.8 | 0.8 |
| Copolymer of acrylate/ dimethicone^{(a)} in linear pentadimethylsiloxane^{(b) (**)} | ^{(a)} 1.2 | ^{(a)} 1.2 | | ^{(a)} 1.2 |
| | ^{(b)} 1.8 | ^{(b)} 1.8 | - | ^{(b)} 1.8 |
| Ethanol | 3 | 3 | 3 | 3 |
| Water | qs 100 | qs 100 | qs 100 | qs 100 |

| | | | | |
|---|---|---|---|---|
| ^{(*)} mixture of Carbopol Ultrez 10 and Carbopol 980 according to the ratio 2/1 ^{(**)} KP-545L sold by the company Shin-Etsu | | | | |

### 2) Procedure

For each of the compositions above, the aqueous phase is prepared by mixing, with mechanical stirring at 65°C, the following ingredients: phenoxyethanol, disodium EDTA, styrene/acrylate copolymer, propanediol and water.

The carbomer and the crosslinked polymer of C₁₀-₃₀ alkyl acrylate are then added and dispersed with mechanical stirring.

The triethanolamine is then added.

In parallel, the oily phase is prepared by mixing, with mechanical stirring at 80°C, the UV-screening agents with glyceryl monooleate or polysorbate 20.

The aqueous and oily phases thus obtained are macroscopically homogeneous.

The composition (A1), according to the invention, and the comparative compositions (B1), (B2) and (B3) in the form of an oil-in-water emulsion are prepared by slow introduction of the fatty phase into the aqueous phase with stirring using a homogenizer of COS 1000 type with a stirring speed of 4000 rpm for 15 minutes. The mixture of the acrylate/dimethicone copolymer and linear pentadimethylsiloxane is then introduced with stirring into the emulsion.

The emulsion thus obtained is cooled with stirring to 40°C, before the addition of the nylon 12 and of the ethanol with gentle stirring.

The emulsions are then cooled to ambient temperature with slow stirring.

They are characterized by oil drops having a size ranging from 500 nanometres to 10 µm. The size of the oil drops was measured by optical microscopy in white light.

The level of anti-sun protection, and also the quality of application to wet skin, of each of the compositions (A1), (B1), (B2) and (B3) were evaluated according to the following methods.

### a. Level of anti-sun protection

The level of anti-sun protection is defined by the sun protection factor (SPF) which is determined according to the *"in vitro"* method described by B. L. Diffey in J. Soc. Cosmet. Chem. 40, 127-133, (1989).

Each composition was applied to a rough plate of PMMA, in the form of a uniform and even deposit in a proportion of 1 mg.cm⁻².

The sun protection factors (SPFs) were measured using a UV-1000S spectrophotometer from the company Labsphere.

### b. Quality of application to wet skin

Each of the compositions obtained above was applied to a substrate, which is a skin substitute.

The skin substitute is an elastomer composed of several layers, an internal layer of silicone and an external layer of polyurethane.

The skin substitute was immersed beforehand in a bath of demineralized water at 28°C for 5 minutes, before the application of the compositions (A1), (B1), (B2) and (B3). Each of the compositions was applied using a micro pipette in a proportion of 2 mg.cm⁻² before being distributed very rapidly and uniformly with a fingerstall.

The obtaining of a uniform film was evaluated with the naked eye.

Moreover, the transparency on the substrate was evaluated by means of a colorimetric measurement of the parameter ΔL (Delta L, difference in luminance between a zone with formula and a zone without formula) using a spectrocolorimeter (CM 2600d from the company Konica Minolta). When the composition is compatible with wet skin, the parameter ΔL is less than 0.6.

In parallel, the compatibility of the compositions with dry skin was evaluated on a dry substrate. The compositions (A1), (B1), (B2) and (B3) were applied using a micro pipette in a proportion of 2 mg.cm⁻². They were then distributed very rapidly and uniformly with a fingerstall. After one minute of drying, the parameter ΔL was measured using a spectrocolorimeter (CM 2600d from the company Konica Minolta).

### 3) Results

The results are given in the table below.

| | Invention A1 | Comparative B1 | Comparative B2 | Comparative B3 |
|---|---|---|---|---|
| SPF *in vitro* | 156±30 | 89±18 | 126±25 | 105±20 |
| Presence of a uniform and transparent deposit on wet skin | +++ | --- | + (heterogeneous film) | --- |
| ΔL on wet skin | 0.31 | 0.72 | Not significant (heterogeneous film) | 0.93 |
| ΔL on dry skin | 0.34 | 1.15 | 0.75 | 1.73 |

The results obtained above show that only the composition (A1) prepared according to the present invention, i.e. comprising an anionic crosslinked hydrophilic polymer, a surfactant having an HLB less than or equal to 5, and a silicone copolymer as claimed, has good UV-screening properties and excellent compatibility with wet skin. Indeed, the deposit is uniform and transparent and the colorimetric parameter ΔL is less than 0.6 (0.31). Moreover, the sun protection factor (SPF) is greater than 130 (156).

The composition (A1) is also stable over time and has good photoprotective properties with an *in vivo* SPF level evaluated at 50.

Conversely, for the comparative compositions (B1), (B2) and (B3) which do not comprise the combination of the invention, the performance levels in terms of screening efficiency and/or of compatibility with wet skin are inferior to those obtained with the composition of the invention (A1).

### EXAMPLE 2

### 1) Preparation of the compositions

The following compositions (A1), (A2) and (A3) were prepared according to the present invention from ingredients of which the contents are indicated in the table below.

The weight ratio R between the amount of silicone copolymer and the amount of anionic crosslinked hydrophilic polymer was calculated for each of the compositions (A1), (A2) and (A3).

| | Invention A1 | Invention A2 | Invention A2 |
|---|---|---|---|
| Triethanolamine | 0.25 | 0.25 | 0.25 |
| Phenoxyethanol | 0.7 | 0.7 | 0.7 |
| Disodium EDTA | 0.05 | 0.05 | 0.05 |
| Styrene/acrylate copolymer | 0.9 | 0.9 | 0.9 |
| Carbomer^{(*)} | 0.3 | 0.3 | 0.3 |
| Propanediol | 3 | 3 | 3 |
| Homosalate | 10 | 10 | 10 |
| Ethylhexyl salicylate | 5 | 5 | 5 |
| Drometrizone trisiloxane | 0.9 | 0.9 | 0.9 |
| Bis(ethylhexylphenol)methoxyphenyltriazine | 1.7 | 1.7 | 1.7 |
| Octocrylene | 5 | 5 | 5 |
| Butyl methoxy dibenzoylmethane | 5 | 5 | 5 |
| Ethylhexyl triazone | 2.35 | 2.35 | 2.35 |
| Glyceryl monooleate | 0.4 | 0.4 | 0.4 |
| Nylon 12 | 0.8 | 0.8 | 0.8 |
| Copolymer of acrylate/ dimethicone^{(a)} in linear pentadimethylsiloxane^{(b) (**)} | ^{(a)} 1.2 | ^{(a)} 1.8 | ^{(a)} 3 |
| | ^{(b)} 1.8 | ^{(b)} 2.7 | ^{(b)} 4.5 |
| Linear pentadimethyl siloxane | 2.7 | 1.8 | - |
| Ethanol | 3 | 3 | 3 |
| Water | qs 100 | qs 100 | qs 100 |
| R | 4 | 6 | 10 |

| | | | |
|---|---|---|---|
| ^{(*)} mixture of Carbopol Ultrez 10 and Carbopol 980 according to the ratio 2/1 ^{(**)} KP-545L sold by the company Shin-Etsu | | | |

### 2) Procedure

The compositions (A1), (A2) and (A3) were prepared according to the same protocol as that previously described.

The tacky effect of each of the compositions (A1), (A2) and (A3) was measured with a Swantech TAXT2 texturometer equipped with Texture expert exceed V212 software.

Each of the compositions (A1), (A2) and (A3) is deposited on a flat support and spread using a film drawer, having a thickness of 100 microns, at 25°C, in order to obtain a film of homogeneous composition.

The tacky effect is then measured using a metal support which moves at a speed of 0.10 mm/s until the film of composition to be tested is achieved. The tacky effect corresponds to the withdrawal force required to detach the film.

### 3) Results

The results are given in the table below.

| | Invention A1 | Invention A2 | Invention A3 |
|---|---|---|---|
| Force (N) | 0.2 | 0.35 | 0.48 |

The results obtained above show that the composition (A1), for which the weight ratio R between the amount of silicone copolymers and the amount of anionic crosslinked hydrophilic polymers is less than 5, has improved cosmetic properties, in particular in terms of tacky-feel effect. It is in fact less tacky to the feel than the compositions (A2) and (A3) for which the weight ratio R is greater than 5.

## Claims

1. Composition in the form of an oil-in-water emulsion comprising:
a) an oily phase, dispersed in a continuous aqueous phase,
b) one or more UV-screening agents chosen from water-soluble organic UV-screening agents, liposoluble organic UV-screening agents, insoluble organic UV-screening agents, and mixtures thereof,
c) one or more anionic crosslinked hydrophilic polymers,
d) one or more surfactants having an HLB less than or equal to 5, and
e) one or more silicone copolymers resulting from the copolymerization of at least one monomer of the type of an ester of a carboxylic acid and of an alcohol comprising a C₆ to C₃₀ fatty chain, with at least one monomer containing a polyalkylsiloxane chain;
the weight ratio between the amount of silicone copolymer(s) and the amount of anionic crosslinked hydrophilic polymer(s) being less than or equal to 5.

2. Composition according to the preceding claim, **characterized in that** the UV-screening agent(s) are chosen from liposoluble organic UV-screening agents, preferably chosen from dibenzoylmethane compounds, salicylic compounds, β,β-diphenylacrylate compounds, benzophenone compounds, phenyl benzotriazole compounds, triazine compounds and mixtures thereof, and more preferentially from homosalate, ethylhexyl salicylate, drometrizole trisiloxane, bis-ethylhexyloxyphenol methoxyphenyl triazine, octocrylene, butyl methoxydibenzoylmethane, ethylhexyl triazone, n-hexyl 2-(4-diethylamino-2-hydroxybenzoyl)benzoate, diethylhexyl butamido triazone and mixtures thereof.

3. Composition according to any one of the preceding claims, **characterized in that** the amount of the UV-screening agent(s) ranges from 0.1% to 50% by weight, preferably from 10% to 50% by weight, more preferentially from 20% to 50% by weight and better still from 20% to 40% by weight, relative to the total weight of the composition.

4. Composition according to any one of the preceding claims, **characterized in that** the anionic crosslinked hydrophilic polymer(s) are chosen from crosslinked 2-acrylamido-2-methylpropanesulfonic acid homo- and copolymers, homo- and copolymers of at least one α,β-ethylenically unsaturated carboxylic acid monomer, and mixtures thereof, preferably from polymers comprising at least one α,β-ethylenically unsaturated carboxylic acid monomer, and more preferentially from crosslinked (meth)acrylic acid homopolymers.

5. Composition according to any one of the preceding claims, **characterized in that** the amount of the anionic crosslinked hydrophilic polymer(s) ranges from 0.05% to 5% by weight, preferably from 0.08% to 2.5% by weight and more preferentially from 0.1% to 1.5% by weight, relative to the total weight of the composition.

6. Composition according to any one of the preceding claims, **characterized in that** the surfactant(s) having an HLB less than or equal to 5 are non-ionic, and are preferably chosen from alkyl and polyalkyl esters of poly(ethylene oxide); oxyalkylenated fatty alcohols; alkyl and polyalkyl ethers of poly(ethylene oxide); polyoxyethylenated or non-polyoxyethylenated alkyl and polyalkyl esters of sorbitan; non-polyoxyethylenated alkyl and polyalkyl ethers of sorbitan; alkyl- and polyalkylglycosides or polyglycosides, in particular alkyl- and polyalkylglucosides or polyglucosides; alkyl and polyalkyl esters of sucrose; polyoxyethylenated or non-polyoxyethylenated alkyl and polyalkyl esters of glycerol; polyoxyethylenated or non-polyoxyethylenated alkyl and polyalkyl ethers of glycerol; and mixtures thereof, these compounds comprising at least one fatty chain comprising from 6 to 30 carbon atoms, preferably from 12 to 30 carbon atoms, and more preferentially from 14 to 24 carbon atoms.

7. Composition according to any one of the preceding claims, **characterized in that** the surfactant(s) having an HLB less than or equal to 5 are chosen from steareth-2, beheneth-2, oleth-2, sucrose distearate, glyceryl monooleate, glyceryl stearate, glyceryl isostearate, diglyceryl distearate, tetraglyceryl tristearate, decaglyceryl decastearate, diglyceryl monostearate, hexaglyceryl tristearate, decaglyceryl pentastearate, sorbitan monostearate, sorbitan tristearate, diethylene glycol monostearate, the ester of glycerol and of palmitic and stearic acids, glyceryl monobehenate, glyceryl dibehenate, and mixtures thereof; and preferably glyceryl monooleate.

8. Composition according to any one of the preceding claims, **characterized in that** the amount of the surfactant(s) having an HLB less than or equal to 5 ranges from 0.1% to 3% by weight, preferably from 0.2% to 2% by weight and more preferentially from 0.3% to 1% by weight, relative to the total weight of the composition.

9. Composition according to any one of the preceding claims, **characterized in that** the monomer(s) of the type ester of carboxylic acid and of alcohol comprising a C₆ to C₃₀ fatty chain are chosen from monomers of the type ester of (meth)acrylic acid and of alcohol comprising a C₆ to C₃₀ fatty chain of structure (XVII) and mixtures thereof 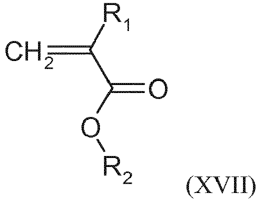 in which
R₁ represents a hydrogen atom or a methyl, and
R₂ represents a linear or branched, saturated or unsaturated C₆ to C₃₀, preferably C₇ to C₂₄, alkyl chain.

10. Composition according to Claim 9, **characterized in that** R₂ represents a branched, saturated or unsaturated C₇ to C₂₄ alkyl chain.

11. Composition according to any one of the preceding claims, **characterized in that** the monomer(s) containing a polyalkylsiloxane chain are chosen from monomers of the type (meth)acrylic acid esterified with a silicone group of structure (XVIII) 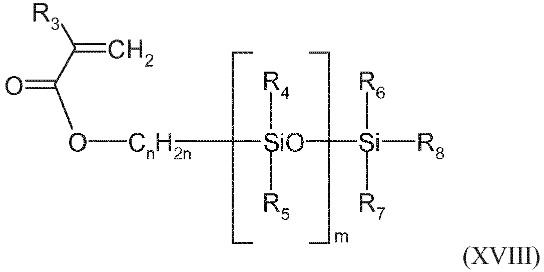 in which
R₃ represents a hydrogen atom or a methyl, and
R₄, R₅, R₆, R₇ and R₈, which may be identical or different, represent, independently of one another, a linear or branched, saturated or unsaturated C₁ to C₃₀ alkyl chain,
n and m represent, independently of each other, an integer greater than or equal to 1.

12. Composition according to any one of the preceding claims, **characterized in that** the silicone copolymer(s) also comprise one or more additional monomers, preferably one or more monomers which are an ester of a carboxylic acid and of an alcohol of C₁ to C₅, and more preferentially chosen from esters of (meth)acrylic acid and of a C₁ to C₄ alcohol and mixtures thereof.

13. Composition according to any one of the preceding claims, **characterized in that** the silicone copolymer(s) are chosen from copolymers of stearyl methacrylate/methyl methacrylate/propyl methacrylate containing a polydimethylsiloxane chain; copolymers of methyl methacrylate/butyl methacrylate/2-ethylhexyl acrylate/propyl methacrylate containing a polydimethylsiloxane chain; and mixtures thereof.

14. Composition according to any one of the preceding claims, **characterized in that** the amount of the silicone copolymer(s) ranges from 0.5% to 10% by weight, preferably from 1% to 8% by weight and more preferentially from 2% to 5% by weight, relative to the total weight of the composition.

15. Composition according to any one of the preceding claims, **characterized in that** the weight ratio between the amount of silicone copolymer(s) and the amount of anionic crosslinked hydrophilic polymer(s) is less than or equal to 4.5, and more preferentially ranges from 1 to 4.5.

16. Composition according to any one of the preceding claims, **characterized in that** it also comprises one or more efficiency boosters, preferably chosen from particles of copolymer of styrene and of acrylates; waxes; gelling lipophilic polymers; and mixtures thereof.

17. Composition according to any one of the preceding claims, **characterized in that** it also comprises one or more insoluble particles.

18. Composition as defined in any one of the preceding claims for its use in treating the skin against UV radiation, in particular against solar radiation.

## Patentansprüche

1. Zusammensetzung in Form einer Öl-in-Wasser-Emulsion, umfassend:
a) eine ölige Phase, die in einer kontinuierlichen wässrigen Phase dispergiert ist,
b) ein oder mehrere UV-Filtersubstanzen, aus wasserlöslichen organischen UV-Filtersubstanzen, fettlöslichen organischen UV-Filtersubstanzen, unlöslichen organischen UV-Filtersubstanzen, und Mischungen davon ausgewählt ist bzw. sind,
c) ein oder mehrere anionische vernetzte hydrophile Polymere,
d) ein oder mehrere Tenside mit einem HLB-Wert kleiner oder gleich 5 und
e) ein oder mehrere Silikoncopolymere, die sich aus der Copolymerisation von mindestens einem Monomer des Typs eines Esters einer Carbonsäure und eines Alkohols mit einer C₆- bis C₃₀-Fettkette mit mindestens einem Monomer mit einer Polyalkylsiloxankette ergeben;
wobei das Gewichtsverhältnis zwischen der Menge von Silikoncopolymer(en) und der Menge von anionischem vernetztem hydrophilem Polymer bzw. anionischen vernetzten hydrophilen Polymeren kleiner oder gleich 5 ist.

2. Zusammensetzung nach dem vorhergehenden Ansprüche, **dadurch gekennzeichnet, dass** die UV-Filtersubstanz bzw. die UV-Filtersubstanzen aus fettlöslichen organischen UV-Filtersubstanzen, vorzugsweise aus Dibenzoylmethan-Verbindungen, Salicylsäure-Verbindungen, β,β-Diphenylacrylat-Verbindungen, Benzophenon-Verbindungen, Phenylbenzotriazol-Verbindungen, Triazin-Verbindungen und Mischungen davon und weiter bevorzugt aus Homosalat, Ethylhexylsalicylat, Drometrizoltrisiloxan, Bisethylhexyloxyphenolmethoxyphenyltriazin, Octocrylen, Butylmethoxydibenzoylmethan, Ethylhexyltriazon, 2-(4-Dimethylamino-2-hydroxybenzoyl)benzoesäure-n-hexylester, Diethylhexylbutamidotriazon und Mischungen davon ausgewählt ist bzw. sind.

3. Zusammensetzung nach einem der vorhergehenden Ansprüche, **dadurch gekennzeichnet, dass** die Menge der UV-Filtersubstanz bzw. der UV-Filtersubstanzen im Bereich von 0,1 bis 50 Gew.-%, vorzugsweise von 10 bis 50 Gew.-%, weiter bevorzugt von 20 bis 50 Gew.-% und noch besser von 20 bis 40 Gew.-%, bezogen auf das Gesamtgewicht der Zusammensetzung, liegt.

4. Zusammensetzung nach einem der vorhergehenden Ansprüche, **dadurch gekennzeichnet, dass** das anionische vernetzte hydrophile Polymer bzw. die anionischen vernetzten hydrophilen Polymere aus vernetzten 2-Acrylamido-2-methylpropansulfonsäure-Homo- und Copolymeren, Homo- und Copolymeren von mindestens einem α,β-ethylenisch ungesättigtens Carbonsäure-Monomer und Mischungen davon, vorzugsweise aus Polymeren, die mindestens ein α,β-ethylenisch ungesättigtes Carbonsäure-Monomer umfassen, und weiter bevorzugt aus vernetzten (Meth)acrylsäure-Homopolymeren ausgewählt ist bzw. sind.

5. Zusammensetzung nach einem der vorhergehenden Ansprüche, **dadurch gekennzeichnet, dass** die Menge des anionischen vernetzten hydrophilen Polymers bzw. der anionischen vernetzten hydrophilen Polymere im Bereich von 0,05 bis 5 Gew.-%, vorzugsweise von 0,08 bis 2,5 Gew.-% und weiter bevorzugt von 0,1 bis 1,5 Gew.-%, bezogen auf das Gesamtgewicht der Zusammensetzung, liegt.

6. Zusammensetzung nach einem der vorhergehenden Ansprüche, **dadurch gekennzeichnet, dass** das Tensid bzw. die Tenside mit einem HLB-Wert kleiner oder gleich 5 nichtionisch ist bzw. sind und vorzugsweise aus Alkyl- und Polyalkylestern von Poly(ethylenoxid); oxyalkylenierten Fettalkoholen; Alkyl- und Polyalkylethern von Poly(ethylenoxid); polyoxyethylenierten oder nicht polyoxyethylenierten Alkyl- und Polyalkylestern von Sorbitan; nicht polyoxyethylenierten Alkyl- und Polyalkylethern von Sorbitan; Alkyl- und Polyalkylglycosiden oder - polyglycosiden, insbesondere Alkyl- und Polyalkylglucosiden oder -polyglucosiden; Alkyl- und Polyalkylestern von Saccharose; polyoxyethylenierten oder nicht polyoxyethylenierten Alkyl- und Polyalkylestern von Glycerin; polyoxyethylenierten oder nicht polyoxyethylenierten Alkyl- und Polyalkylethern von Glycerin und Mischungen davon, wobei diese Verbindungen mindestens eine Fettkette mit 6 bis 30 Kohlenstoffatomen, vorzugsweise 12 bis 30 Kohlenstoffatomen und weiter bevorzugt 14 bis 24 Kohlenstoffatomen umfassen, ausgewählt ist bzw. sind.

7. Zusammensetzung nach einem der vorhergehenden Ansprüche, **dadurch gekennzeichnet, dass** das Tensid bzw. die Tenside mit einem HLB-Wert kleiner oder gleich 5 aus Steareth-2, Beheneth-2, Oleth-2, Saccharosedistearat, Glycerylmonooleat, Glycerylstearat, Glycerylisostearat, Diglyceryldistearat, Tetraglyceryltristearat, Decaglyceryldecastearat, Diglycerylmonostearat, Hexaglyceryltristearat, Decaglycerylpentastearat, Sorbitanmonostearat, Sorbitantristearat, Diethylenglykolmonostearat, dem Ester von Glycerin und Palmitinsäure und Stearinsäure, Glycerylmonobehenat, Glyceryldibehenat und Mischungen davon und vorzugsweise Glycerylmonooleat ausgewählt ist bzw. sind.

8. Zusammensetzung nach einem der vorhergehenden Ansprüche, **dadurch gekennzeichnet, dass** die Menge des Tensids bzw. der Tenside mit einem HLB-Wert kleiner oder gleich 5 im Bereich von 0,1 bis 3 Gew.-%, vorzugsweise von 0,2 bis 2 Gew.-% und weiter bevorzugt von 0,3 bis 1 Gew.-%, bezogen auf das Gesamtgewicht der Zusammensetzung, liegt.

9. Zusammensetzung nach einem der vorhergehenden Ansprüche, **dadurch gekennzeichnet, dass** das Monomer bzw. die Monomere des Typs Ester von Carbonsäure und von Alkohol mit einer C₆- bis C₃₀-Fettkette aus Monomeren des Typs Ester von (Meth)acrylsäure und von Alkohol mit einer C₆- bis C₃₀-Fettkette der Struktur (XVII) und Mischungen davon ausgewählt ist bzw. sind: 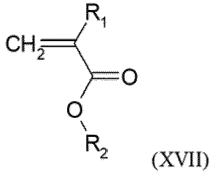 wobei
R₁ für ein Wasserstoffatom oder ein Methyl steht und
R₂ für eine lineare oder verzweigte, gesättigte oder ungesättigte C₆- bis C₃₀- und vorzugsweise C₇- bis C₂₄-Alkylkette steht.

10. Zusammensetzung nach Anspruch 9, **dadurch gekennzeichnet, dass** R₂ für eine verzweigte, gesättigte oder ungesättigte C₇- bis C₂₄-Alkylkette steht.

11. Zusammensetzung nach einem der vorhergehenden Ansprüche, **dadurch gekennzeichnet, dass** das Monomer bzw. die Monomere mit einer Polyalkylsiloxankette aus Monomeren des Typs mit einer Silikongruppe veresterte (Meth)acrylsäure der Struktur (XVIII) ausgewählt ist bzw. sind: 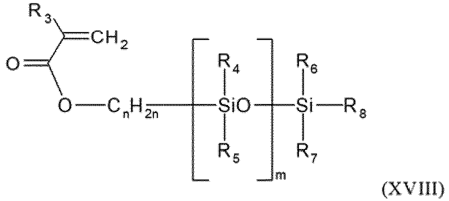 wobei
R₃ für ein Wasserstoffatom oder ein Methyl steht und
R₄, R₅, R₆, R₇ und R₈, die gleich oder verschieden sein können, unabhängig voneinander für eine lineare oder verzweigte, gesättigte oder ungesättigte C₁-bis C₃₀-Alkylkette stehen,
n und m unabhängig voneinander für eine ganze Zahl größer oder gleich 1 stehen.

12. Zusammensetzung nach einem der vorhergehenden Ansprüche, **dadurch gekennzeichnet, dass** das Silikoncopolymer bzw. die Silikoncopolymere außerdem ein oder mehrere zusätzliche Monomere, vorzugsweise ein oder mehrere Monomere, bei denen es sich um einen Ester einer Carbonsäure und eines C₁- bis C₅-Alkohols handelt und die weiter bevorzugt aus Estern von (Meth)acrylsäure und einem C₁- bis C₄-Alkohol und Mischungen davon ausgewählt sind, umfasst bzw. umfassen.

13. Zusammensetzung nach einem der vorhergehenden Ansprüche, **dadurch gekennzeichnet, dass** das Silikoncopolymer bzw. die Silikoncopolymere aus Copolymeren von Stearylmethacrylat/Methylmethacrylat/Propylmethacrylat mit einer Polydimethylsiloxankette; Copolymeren von Methylmethacrylat/Butylmethacrylat/2-Ethylhexylacrylat/Propylmethacrylat mit einer Polydimethylsiloxankette und Mischungen davon ausgewählt ist bzw. sind.

14. Zusammensetzung nach einem der vorhergehenden Ansprüche, **dadurch gekennzeichnet, dass** die Menge des Silikoncopolymers bzw. der Silikoncopolymere im Bereich von 0,5 bis 10 Gew.-%, vorzugsweise von 1 bis 8 Gew.-% und weiter bevorzugt von 2 bis 5 Gew.-%, bezogen auf das Gesamtgewicht der Zusammensetzung, liegt.

15. Zusammensetzung nach einem der vorhergehenden Ansprüche, **dadurch gekennzeichnet, dass** das Gewichtsverhältnis zwischen der Menge von Silikoncopolymer(en) und der Menge von anionischem vernetztem hydrophilem Polymer bzw. anionischen vernetzten hydrophilen Polymeren kleiner oder gleich 4,5 ist und weiter bevorzugt im Bereich von 1 bis 4,5 liegt.

16. Zusammensetzung nach einem der vorhergehenden Ansprüche, **dadurch gekennzeichnet, dass** sie außerdem einen oder mehrere Effizienzverstärker umfasst, die vorzugsweise aus Teilchen von Styrol-Acrylat-Copolymer; Wachsen; gelierenden lipophilen Polymeren und Mischungen davon ausgewählt sind.

17. Zusammensetzung nach einem der vorhergehenden Ansprüche, **dadurch gekennzeichnet, dass** sie außerdem ein oder mehrere unlösliche Teilchen umfasst.

18. Zusammensetzung gemäß einem der vorhergehenden Ansprüche zur Verwendung bei der Behandlung der Haut gegen UV-Strahlung, insbesondere gegen Sonnenstrahlung.

## Revendications

1. Composition sous forme d'une émulsion huile-dans-eau comprenant :
a) une phase huileuse, dispersée dans une phase aqueuse continue,
b) un ou plusieurs filtres UV choisis parmi les filtres UV organiques hydrosolubles, les filtres UV organiques liposolubles, les filtres UV organiques insolubles, et leurs mélanges,
c) un ou plusieurs polymères hydrophiles réticulés anioniques,
d) un ou plusieurs tensioactifs ayant une HLB inférieure ou égale à 5, et
e) un ou plusieurs copolymères siliconés résultant de la copolymérisation d'au moins un monomère de type ester d'acide carboxylique et d'alcool à chaîne grasse en C₆ à C₃₀, avec au moins un monomère contenant une chaîne polyalkylsiloxane ;
le rapport pondéral entre la quantité de copolymère(s) siliconé(s) et la quantité de polymère(s) hydrophile(s) réticulé(s) anionique(s) étant inférieur ou égal à 5.

2. Composition selon la revendication précédente, **caractérisée en ce que** le ou les filtres UV sont choisis parmi les filtres UV organiques liposolubles; de préférence choisis parmi les composés dibenzoylméthanes, les composés salicyliques, les composés β,β-diphénylacrylate, les composés benzophénone, les composés phényl benzotriazole, les composés triazine et leurs mélanges ; et plus préférentiellement parmi l'homosalate, l'éthylhéxyl salicylate, le drométrizole trisiloxane, le bis-éthylhéxyloxyphénol méthoxyphényl triazine, l'octocrylène, le butyl méthoxydibenzoylméthane, l'éthylhéxyl triazone, le 2-(4-diethylamino-2-hydroxybenzoyl)-benzoate de n-hexyle, le diéthylhexyl butamido triazone et leurs mélanges.

3. Composition selon l'une quelconque des revendications précédentes, **caractérisée en ce que** la quantité du ou des filtres UV va de 0,1 à 50% en poids, de préférence de 10 à 50% en poids, plus préférentiellement de 20 à 50% en poids, et mieux encore de 20 à 40% en poids, par rapport au poids total de la composition.

4. Composition selon l'une quelconque des revendications précédentes, **caractérisée en ce que** le ou les polymères hydrophiles réticulés anioniques sont choisis parmi les homo- et co-polymères d'acide 2-acrylamido 2-méthylpropane sulfonique réticulés, les homo- et co-polymères d'au moins un monomère acide carboxylique à insaturation α,β-éthylénique, et leurs mélanges, de préférence parmi les polymères comprenant au moins un monomère acide carboxylique à insaturation α,β-éthylénique, et plus préférentiellement parmi les homopolymères d'acide (méth)acrylique réticulés.

5. Composition selon l'une quelconque des revendications précédentes, **caractérisée en ce que** la quantité du ou des polymères hydrophiles réticulés anioniques va de 0,05 à 5% en poids, de préférence de 0,08 à 2,5% en poids, et plus préférentiellement de 0,1 à 1,5% en poids, par rapport au poids total de la composition.

6. Composition selon l'une quelconque des revendications précédentes, **caractérisée en ce que** le ou les tensioactifs ayant une HLB inférieure ou égale à 5 sont non ioniques, et sont de préférence choisis parmi les alkyl- et polyalkyl- esters de poly(oxyde d'éthylène) ; les alcools gras oxyalkylénés ; les alkyl- et polyalkyl-éthers de poly(oxyde d'éthylène) ; les alkyl- et polyalkyl-esters de sorbitane, polyoxyéthylénés ou non polyoxyéthylénés ; les alkyl- et polyalkyl-éthers de sorbitane non polyoxyéthylénés ; les alkyl- et polyalkyl-glycosides ou polyglycosides, en particulier les alkyl- et polyalkyl-glucosides ou polyglucosides ; les alkyl- et polyalkyl-esters de sucrose ; les alkyl- et polyalkyl-esters de glycérol, polyoxyéthylénés ou non ; les alkyl- et polyalkyl-éthers de glycérol, polyoxyéthylénés ou non ; et leurs mélanges, ces composés comprenant au moins une chaîne grasse comportant de 6 à 30 atomes de carbone, de préférence de 12 à 30 atomes de carbone, et plus préférentiellement de 14 à 24 atomes de carbone.

7. Composition selon l'une quelconque des revendications précédentes, **caractérisée en ce que** le ou les tensioactifs ayant une HLB inférieure ou égale à 5 sont choisis parmi le stéareth-2, le beheneth-2, l'oleth-2, le distéarate de sucrose, le mono oléate de glycéryle, le stéarate de glycéryle, l'isostéarate de glycéryle, le distéarate de diglycéryle, le tristéarate de tétraglycéryle, le décastéarate de décaglycéryle, le monostéarate de diglycéryle, le tristéarate d'hexaglycéryle, le pentastéarate de décaglycéryle, le monostéarate de sorbitane, le tristéarate de sorbitane, le monostéarate de diéthylène glycol, l'ester de glycérol et d'acides palmitique et stéarique, le monobéhénate de glycéryle, le dibéhénate de glycéryle, et leurs mélanges, et de préférence le mono oléate de glycérol.

8. Composition selon l'une quelconque des revendications précédentes, **caractérisée en ce que** la quantité du ou des tensioactifs ayant une HLB inférieure ou égale à 5 va de 0,1 à 3% en poids, de préférence de 0,2 à 2% en poids, et plus préférentiellement de 0,3 à 1% en poids, par rapport au poids total de la composition.

9. Composition selon l'une quelconque des revendications précédentes, **caractérisée en ce que** le ou les monomères de type ester d'acide carboxylique et d'alcool à chaîne grasse en C₆ à C₃₀ sont choisis parmi les monomères de type ester d'acide (méth)acrylique et d'alcool à chaîne grasse en C₆ à C₃₀ de structure (XVII) et leurs mélanges 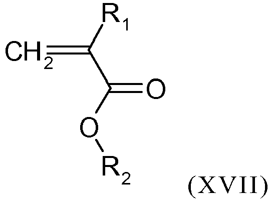 dans laquelle,
R₁ représente un atome d'hydrogène ou un méthyle, et
R₂ représente une chaîne alkyle linéaire ou ramifiée, saturée ou insaturée, en C₆ à C₃₀, de préférence en C₇ à C₂₄.

10. Composition selon la revendication 9, **caractérisée en ce que** R₂ représente une chaîne alkyle ramifiée, saturée ou insaturée, en C₇ à C₂₄.

11. Composition selon l'une quelconque des revendications précédentes, **caractérisée en ce que** le ou les monomères contenant une chaîne polyalkylsiloxane sont choisis parmi les monomères de type acide (méth)acrylique estérifié par un groupement siliconé de structure (XVIII) 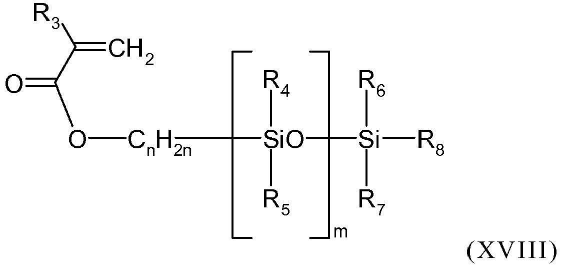 dans laquelle,
R₃ représente un atome d'hydrogène ou un méthyle, et
R₄, R₅, R₆, R₇ et R₈, identiques ou différents, représentent, indépendamment les uns des autres, une chaîne alkyle linéaire ou ramifiée, saturée ou insaturée, en C₁ à C₃₀,
n et m représentent indépendamment l'un de l'autre un nombre entier supérieur ou égal à 1.

12. Composition selon l'une quelconque des revendications précédentes, **caractérisée en ce que** le ou les copolymères siliconés comprennent en outre un ou plusieurs monomères additionnels, de préférence un ou plusieurs monomères ester d'acide carboxylique et d'alcool en C₁ à C₅, et plus préférentiellement choisis parmi les esters d'acide (méth)acrylique et d'alcool en C₁ à C₄ et leurs mélanges.

13. Composition selon l'une quelconque des revendications précédentes, **caractérisée en ce que** le ou les copolymères siliconés sont choisis parmi les copolymères de méthacrylate de stéaryle, de méthacrylate de méthyle, et de méthacrylate de propyle contenant une chaîne polydiméthylsiloxane ; les copolymères de méthacrylate de méthyle, de méthacrylate de butyle, d'acrylate de 2-éthylhéxyle et de méthacrylate de propyle contenant une chaîne polydiméthylsiloxane ; et leurs mélanges.

14. Composition selon l'une quelconque des revendications précédentes, **caractérisée en ce que** la quantité du ou des copolymères siliconés va de 0,5 à 10% en poids, de préférence de 1 à 8% en poids, et plus préférentiellement de 2 à 5% en poids, par rapport au poids total de la composition.

15. Composition selon l'une quelconque des revendications précédentes, **caractérisée en ce que** le rapport pondéral entre la quantité de copolymère(s) siliconé(s) et la quantité de polymère(s) hydrophile(s) réticulé(s) anionique(s) est inférieur ou égal à 4,5, et plus préférentiellement va de 1 à 4,5.

16. Composition selon l'une quelconque des revendications précédentes, **caractérisée en ce qu'**elle comprend en outre un ou plusieurs boosters d'efficacité, de préférence choisis parmi les particules de copolymère de styrène et d'acrylates ; les cires ; les polymères lipophiles gélifiants ; et leurs mélanges.

17. Composition selon l'une quelconque des revendications précédentes, **caractérisée en ce qu'**elle comprend en outre une ou plusieurs particules insolubles.

18. Composition selon l'une quelconque des revendications précédentes pour son utilisation dans le traitement de la peau contre le rayonnement UV, en particulier contre le rayonnement solaire.
